# EUROPEAN PATENT SPECIFICATION

(11) **EP 2 419 736 B1**
(45) Date of publication and mention of the grant of the patent: **29.01.2014**
(21) Application number: 10715065.8
(22) Date of filing: 16.04.2010
(51) Int. Cl.: G01N 33/574, A61K 31/715, G01N 33/50

(54) **METHODS OF ASSESSING ACTIVITY OF A POLYSACCHARIDE COMPOSITION**
VERFAHREN ZUR BEURTEILUNG DER AKTIVITÄT EINER POLYSACCHARIDZUSAMMENSETZUNG
PROCÉDÉ D'ÉVALUATION DE L'ACTIVITÉ D'UNE COMPOSITION DE POLYSACCHARIDE

(30) Priority: 16.04.2009 US 170049 P
(43) Date of publication of application: 22.02.2012
(73) Proprietor: Momenta Pharmaceuticals, Inc., Cambridge, MA 02142 (US)
(72) Inventor: ZHOU, He, Malden, MA 02148 (US); DUFFNER, Jay, Shirley,MA 01464 (US); GALCHEVA-GARGOVA, Zoya, Quincy,MA 02169 (US); KISHIMOTO, Takashi, Kei, Lexington,MA 02420 (US); SCHULTES, Birgit, Arlington, MA 02476 (US)
(74) Representative: Simpson, Tobias Rutger
(86) International application number: PCT/US2010/031480
(87) International publication number: WO 2010/121196

(56) References cited:
- US-A1- 2008 051 567
- DIAZ-MONTERO C MARCELA ET AL: "Increased circulating myeloid-derived suppressor cells correlate with clinical cancer stage, metastatic tumor burden, and doxorubicin-cyclophosphamide chemotherapy" CANCER IMMUNOLOGY IMMUNOTHERAPY, vol. 58, no. 1, January 2009 (2009-01), pages 49-59, XP002588068 ISSN: 0340-7004
- KRAGH MICHAEL ET AL: "Non-anti-coagulant heparins: A promising approach for prevention of tumor metastasis - (Review)" INTERNATIONAL JOURNAL OF ONCOLOGY, DEMETRIOS A. SPANDIDOS ED. & PUB, GR, vol. 27, no. 4, 1 October 2005 (2005-10-01), pages 1159-1167, XP008123477 ISSN: 1019-6439
- OSTRAND-ROSENBERG SUZANNE ET AL: "Myeloid-Derived Suppressor Cells: Linking Inflammation and Cancer" JOURNAL OF IMMUNOLOGY, vol. 182, no. 8, 15 April 2009 (2009-04-15), pages 4499-4506, XP002588069 ISSN: 0022-1767
- GABRILOVICH DMITRY I ET AL: "Myeloid-derived suppressor cells as regulators of the immune system" NATURE REVIEWS IMMUNOLOGY, vol. 9, no. 3, March 2009 (2009-03), pages 162-174, XP002588070
- DE LORENZO FERRUCCIO ET AL: "The role of anticoagulation in cancer patients: Facts and figures" ANTI-CANCER AGENTS IN MEDICINAL CHEMISTRY, vol. 6, no. 6, November 2006 (2006-11), pages 579-587, XP008123475 ISSN: 1871-5206
- GEROTZIAFAS G T ET AL: "Clinical studies with anticoagulants to improve survival in cancer patients" PATHOPHYSIOLOGY OF HAEMOSTASIS AND THROMBOSIS 2008 S. KARGER AG CHE LNKD- DOI:10.1159/000175158, vol. 36, no. 3-4, 2008, pages 204-211, XP008123478 ISSN: 1424-8832
- MOUSA SHAKER A: "Role of current and emerging antithrombotics in thrombosis and cancer" DRUGS OF TODAY, vol. 42, no. 5, May 2006 (2006-05), pages 331-350, XP002588071 ISSN: 1699-3993
- FERRO VITO ET AL: "PI-88 and novel heparan sulfate mimetics inhibit angiogenesis" SEMINARS IN THROMBOSIS AND HEMOSTASIS, vol. 33, no. 5, July 2007 (2007-07), pages 557-562, XP008123474 ISSN: 0094-6176

## Description

### BACKGROUND

Heparin, a highly sulfated heparin-like glycosaminoglycan (HLGAG) produced by mast cells and isolated from natural sources, is a widely used clinical anticoagulant. However, the effects of natural, or unfractionated, heparin can be difficult to predict and patients must be monitored closely to prevent over- or under-anticoagulation. Low molecular weight heparins (LMWHs) obtained by various methods of fractionation or depolymerization of polymeric heparin have more predictable pharmacological action as anticoagulants, reduced side effects, sustained antithrombotic activity, and better bioavailability than unfractionated heparin (UFH). Several LMWHs are approved for outpatient treatment of thrombotic conditions.

There is increasing interest in the potential role of antithrombotic agents in the management of cancer patients.

### SUMMARY OF THE INVENTION

According to the present invention, there is provided a method of monitoring the effect of a polysaccharide preparation on a subject having cancer, the method comprising determining tumor stromal levels of myeloid derived suppressor cells (MDSCs), endothelial progenitor cells (EPCs), plasma matrix metallopeptidase 9 (MMP-9) expression, granulocyte-colony stimulating factor (G-CSF) expression, monokine induced by interferon gamma (MIG) expression or combinations thereof in a sample obtained from the subject after the subject has been administered the polysaccharide preparation, to thereby determine the effect of the polysaccharide preparation on the subject;
wherein the polysaccharide preparation has the following characteristics:
a weight average chain molecular weight between 3,500 and 8,000 Da;
anti-Xa activity and anti-IIa activity each less than 50 IU/mg;
between 5% and 50% glycol split uronic acid residues; and
a molecular weight distribution such that 10-50% of the oligosaccharides of the preparation have a molecular weight < 3000 Da, 40-65% of the oligosaccharides have a molecular weight between 3000-8000 Da, and 5-30% of the oligosaccharides have a molecular weight > 8000 Da;
and wherein a decrease in the level of MDSCs, the level of EPCs, expression level of plasma MMP-9, expression level of G-CSF, or combinations thereof relative to a reference standard indicates that the polysaccharide preparation is effective in treating the cancer in the subject; and an increase or no significant change in the level of MDSCs, the level of EPCs, expression level of plasma MMP-9, expression level of G-CSF, or combinations thereof relative to a reference standard indicates that the polysaccharide preparation is not effective in treating the cancer in the subject.

More generally, the present disclosure features a method of monitoring the effect of a polysaccharide preparation described herein on a subject having a disorder described herein, e.g., cancer, e.g., a cancer described herein, the method comprising:
selecting a subject that has been administered one or more of the polysaccharide preparations described herein; and
determining myeloid derived suppressor cell (MDSC) levels, endothelial progenitor cell (EPC) levels, plasma matrix metallopeptidase 9 (MMP-9) expression levels, granulocyte-colony stimulating factor (G-CSF) expression levels, monokine induced by gamma interferon (MIG) expression levels, or combinations thereof in the subject, to thereby determine the effect of the polysaccharide preparation on the subject.

In some instances, a decrease, e.g., a statistically significant decrease, in the level of MDSCs, the level of EPCs in the blood, expression level of plasma MMP-9, expression level of G-CSF, or combinations thereof relative to a reference standard indicates that the polysaccharide preparation is effective in treating the disorder in the subject. In some instances, an increase or no significant change, e.g., no statistically significant change, in the level of MDSCs, the level of EPCs in the blood, expression level of plasma MMP-9, expression level of G-CSF, or combinations thereof relative to a reference standard indicates that the polysaccharide preparation is not effective in treating the disorder in the subject. In some instances, an increase in the expression level of MIG relative to a reference standard indicates that the polysaccharide preparation is effective in treating the disorder in the subject. In some instances, a decrease or no significant change, e.g., no statistically significant change, in the expression level of MIG relative to a reference standard indicates that the polysaccharide preparation is not effective in treating the disorder in the subject. In some instances, the reference standard is the MDSC level, the EPC level, plasma MMP-9 expression level, G-CSF expression level and/or MIG expression level in the subject prior to administration of the polysaccharide preparation. In some instances, MDSC level, the EPC level, plasma MMP-9 expression level, G-CSF expression level and/or MIG expression level are determined from a sample, e.g., a blood or tissue sample, e.g., a biopsy sample, obtained from the subject. In some instances, MDSC level, EPC level, plasma MMP-9 expression level, G-CSF expression level and/or MIG expression level are determined by a method selected from the group consisting of enzyme-linked immunosorbent assay (ELISA), a radioimmunoassay (RIA), a Western blot, or an immunohistochemical assay (IHC). In some instances, the subject has not previously been administered the polysaccharide preparation or the polysaccharide preparation has previously been administered to the subject on one or more occasions.

Also disclosed herein is a method of monitoring the effect of a polysaccharide preparation described herein on a subject having a disorder described herein, e.g., cancer, e.g., a cancer described herein, the method comprising:
determining myeloid derived suppressor cell (MDSC) levels, endothelial progenitor cell (EPC) levels, plasma matrix metallopeptidase 9 (MMP-9) expression levels, granulocyte-colony stimulating factor (G-CSF) expression levels, monokine induced by gamma interferon (MIG) expression levels, or combinations thereof in the subject,
administering one or more polysaccharide preparations described herein to the subject; and
determining myeloid derived suppressor cell (MDSC) levels, endothelial progenitor cell (EPC) levels, plasma matrix metallopeptidase 9 (MMP-9) expression levels, granulocyte-colony stimulating factor (G-CSF) expression levels, monokine induced by gamma interferon (MIG) expression levels, or combinations thereof in the subject, to thereby determine the effect of the polysaccharide preparation on the subject.

Also disclosed herein is a method of measuring tumor burden in a subject having cancer, e.g., breast cancer (e.g., locally advanced or metastatic breast cancer), melanoma (e.g., metastatic melanoma), colorectal cancer (e.g., locally advanced or metastatic colorectal cancer), pancreatic cancer (e.g., locally advanced or metastatic pancreatic cancer), the method comprising:
determining myeloid derived suppressor cell (MDSC) levels, endothelial progenitor cell (EPC) levels, plasma matrix metallopeptidase 9 (MMP-9) expression levels, granulocyte-colony stimulating factor (G-CSF) expression levels, or combinations thereof in the subject, wherein a decrease, e.g., a statistically significant decrease, in MDSC levels, EPC levels, MMP-9 expression levels, or G-CSF expression levels in the subject is indicative of an improvement in tumor load, e.g., the number of cancer cells, the size of a tumor, and/or the amount of cancer in the body has remain unchanged or has decreased, to thereby determine tumor burden.

In some instances, a decrease, e.g., a statistically significant decrease, in the level of MDSCs, the level ofEPCs, expression level of MMP-9, or expression level of G-CSF relative to a reference standard indicates an improvement in tumor load, e.g., the number of cancer cells, the size of a tumor, and/or the amount of cancer in the body has remain unchanged or has decreased, to thereby determine tumor burden. In some instances, the reference standard is MDSC levels, EPC levels, MMP-9 expression levels, or G-CSF expression levels in the subject prior to administration of a treatment, e.g., wherein the treatment is selected from the group consisting of a polysaccharide preparation described herein, surgical treatment, radiation therapy, chemotherapy, antibody therapy, and hormonal therapy. In some instances, MDSC levels, EPC expression levels, MMP-9 expression levels, or G-CSF expression levels are determined from a sample, e.g., a blood or tissue sample, e.g., a biopsy sample, obtained from the subject. In some instances, MDSC levels, EPC expression levels, MMP-9 expression levels, or G-CSF expression levels are determined by a method selected from the group consisting of enzyme-linked immunosorbent assay (ELISA), a radioimmunoassay (RIA), a Western blot, or an immunohistochemical assay (IHC).

Also disclosed herein is a method of measuring tumor burden in a subject having cancer, e.g., breast cancer (e.g., locally advanced or metastatic breast cancer), melanoma (e.g., metastatic melanoma), colorectal cancer (e.g., locally advanced or metastatic colorectal cancer), pancreatic cancer (e.g., locally advanced or metastatic pancreatic cancer), the method comprising:
determining myeloid derived suppressor cell (MDSC) levels, endothelial progenitor cell (EPC) levels, plasma matrix metallopeptidase 9 (MMP-9) expression levels, granulocyte-colony stimulating factor (G-CSF) expression levels, or combinations thereof in the subject,
providing the subject with a treatment the treatment, e.g., a treatment selected from the group consisting of a polysaccharide preparation described herein, surgical treatment, radiation therapy, chemotherapy, antibody therapy, and hormonal therapy; and
comparing MDSC levels, EPC levels, MMP-9 expression levels, G-CSF expression levels, or combinations thereof in the subject, after the treatment to MDSC levels, EPC levels, MMP-9 expression levels, G-CSF expression levels, or combinations thereof, prior to the treatment to thereby determine the effect of the treatment on tumor load.

Also disclosed herein is a method of measuring tumor burden in a subject having cancer, e.g., breast cancer (e.g., locally advanced or metastatic breast cancer), melanoma (e.g., metastatic melanoma), colorectal cancer (e.g., locally advanced or metastatic colorectal cancer), pancreatic cancer (e.g., locally advanced or metastatic pancreatic cancer), the method comprising:
determining monokine induced by gamma interferon (MIG) expression levels in the subject, wherein an increase, e.g., a statistically significant increase, in MIG expression levels in the subject is indicative of an improvement in tumor load, e.g., the number of cancer cells, the size of a tumor, and/or the amount of cancer in the body has remain unchanged or has decreased, to thereby determine tumor burden.

In some instances, an increase, e.g., a statistically significant increase, in the expression level of MIG relative to a reference standard indicates an improvement in tumor load, e.g., the number of cancer cells, the size of a tumor, and/or the amount of cancer in the body has remain unchanged or has decreased, to thereby determine tumor burden. In some instances, the reference standard is MIG expression levels in the subject prior to administration of a treatment, e.g., wherein the treatment is selected from the group consisting of a polysaccharide preparation described herein, surgical treatment, radiation therapy, chemotherapy, antibody therapy, and hormonal therapy. In some instances, MIG expression levels are determined from a sample, e.g., a blood or tissue sample, e.g., a biopsy sample, obtained from the subject. In some instances, MIG expression levels are determined by a method selected from the group consisting of enzyme-linked immunosorbent assay (ELISA), a radioimmunoassay (RIA), a Western blot, or an immunohistochemical assay (IHC).

Also disclosed herein is a method of measuring tumor burden in a subject having cancer, e.g., breast cancer (e.g., locally advanced or metastatic breast cancer), melanoma (e.g., metastatic melanoma), colorectal cancer (e.g., locally advanced or metastatic colorectal cancer), pancreatic cancer (e.g., locally advanced or metastatic pancreatic cancer), the method comprising:
determining monokine induced by gamma interferon (MIG) expression levels in the subject,
providing the subject with a treatment, e.g., a treatment selected from the group consisting of a polysaccharide preparation described herein; and
comparing MIG expression levels in the subject, after the treatment to MIG expression levels prior to the treatment to thereby determine the effect of the treatment on tumor load.

Also disclosed herein is a method of determining if a subject having cancer, e.g., breast cancer (e.g., locally advanced or metastatic breast cancer), melanoma (e.g., metastatic melanoma), colorectal cancer (e.g., locally advanced or metastatic colorectal cancer), pancreatic cancer (e.g., locally advanced or metastatic pancreatic cancer) is at risk for relapse or becoming refractory or resistant to a polysaccharide preparation described herein, the method comprising:
determining monokine induced by gamma interferon (MIG) expression levels in the subject, wherein an increase, e.g., a statistically significant increase, in MIG expression levels in the subject indicates a decreased risk for relapse and/or development of resistance or becoming refractory to a polysaccharide preparation described herein, to thereby determine risk.

In some instances, an increase, e.g., a statistically significant increase, in the expression level of MIG relative to a reference standard indicates a decreased risk for relapse and/or development of resistance or becoming refractory to a polysaccharide preparation described herein, to thereby determine risk. In some instances, the reference standard is MIG expression levels in the subject prior to administration of a polysaccharide preparation described herein. In some instances, MIG expression levels are determined from a sample, e.g., a blood or tissue sample, e.g., a biopsy sample, obtained from the subject. In some instances, MIG expression levels are determined by a method selected from the group consisting of enzyme-linked immunosorbent assay (ELISA), a radioimmunoassay (RIA), a Western blot, or an immunohistochemical assay (IHC).

Also disclosed herein is a method of determining if a subject having cancer, e.g., breast cancer (e.g., locally advanced or metastatic breast cancer), melanoma (e.g., metastatic melanoma), colorectal cancer (e.g., locally advanced or metastatic colorectal cancer), pancreatic cancer (e.g., locally advanced or metastatic pancreatic cancer) is at risk for relapse or becoming refractory or resistant to a polysaccharide preparation described herein, the method comprising:
determining monokine induced by gamma interferon (MIG) expression levels in the subject, wherein a decrease, e.g., a statistically significant decrease, or no significant change, e.g., no statistically significant change, in MIG expression levels in the subject indicates an increased risk for relapse and/or development of resistance or becoming refractory to a polysaccharide preparation described herein, to thereby determine risk.

In some instances, a decrease, e.g., a statistically significant decrease, or no significant change, e.g., no statistically significant change, in the expression level of MIG relative to a reference standard indicates an increased risk for relapse and/or development of resistance or becoming refractory to a polysaccharide preparation described herein, to thereby determine risk. In some instances, the reference standard is MIG expression levels in the subject prior to administration of a polysaccharide preparation described herein. In some instances, MIG expression levels are determined from a sample, e.g., a blood or tissue sample, e.g., a biopsy sample, obtained from the subject. In some instances, MIG expression levels are determined by a method selected from the group consisting of enzyme-linked immunosorbent assay (ELISA), a radioimmunoassay (RIA), a Western blot, or an immunohistochemical assay (IHC).

In one instance, the polysaccharide preparations are preparations of polysaccharides derived from heparin, that lack substantial anticoagulant activity (e.g., preparations of polysaccharides that have substantially no anticoagulant activity or residual anticoagulant levels) but retain activity in other non-coagulation mediated biological processes, and methods to produce them. These compounds can have one or more of the following features: 1) an anti-Xa activity and an anti-IIa activity each less than 50 IU/mg (e.g., an anti-Xa activity of less than 50 IU/mg, 40 IU/mg, 30 IU/mg or 20 IU/mg but greater than 0.5 IU/mg, 1 IU/mg or 2 IU/mg and/or an anti-IIa activity of less than 50 IU/mg, 40 IU/mg, 30 IU/mg or 20 IU/mg but greater than 0.5 IU/mg, 1 IU/mg or 2 IU/mg), and 2) anti-metastatic, anti-angiogenic, anti-fibrotic and/or anti-inflammatory activity. The polysaccharides disclosed herein can also have structural characteristics that distinguish them from other polysaccharides, (e.g., from commercially available heparins). For example, a polysaccharide preparation provided herein can have one or more of the following characteristics: the preparation has less than 50%, 40%, 30% or 20% glycol split uronic acid residues; the preparation has no more than 3 glycol split uronic acid residues (U_{G}) per polysaccharide chain (e.g., 3, 2 or 1 U_{G} per polysaccharide chain); the preparation has greater than 40% U_{2S}H_{NS,6S} (e.g., greater than 50%, 60%, 70% or 80%) disaccharide residues; degree of desulfation of the preparation is less than 40% (e.g., less than 30%, 20%, 10%); one or more polysaccharide chains in the preparation have a 4,5-unsaturation of a non-reducing end uronic acid residue; one or more polysaccharide chains in the preparation have a 2,5-anhydromannitol residue at the reducing end (e.g., about 10%, 20%, 30%, 40%, 50%, 60%, 70%, 80%, 90% or more or the polysaccharide chains in the preparation have a 2,5-anhydromannitol residue at the reducing end); the weight average molecular weight of the preparation is between 3,500 and 7,000 Da (e.g., 4,300 to 7,000 Da; 4,500 to 7,000 Da, 5,000 to 7,000 Da); and a molecular weight distribution described herein. This disclosure includes preparations having one or more of these properties and characteristics as well as methods of making and using such preparations. The disclosure also features methods of using such preparations.

Accordingly, the polysaccharide preparation (e.g., a heparin-derived preparation) has the following characteristics: (a) a weight average chain molecular weight between 3,500 and 7,000 Da (e.g., 4,300 to 7,000 Da; 4,500 to 7,000 Da, 5,000 to 7,000 Da); (b) an anti-Xa activity and an anti-IIa activity each less than 50 IU/mg (e.g., an anti-Xa activity less than about 40 IU/mg, 30 IU/mg, 20 IU/mg, 15 IU/mg, 10 IU/mg, 5 IU/mg, 4 IU/mg, 3 IU/mg, 2 IU/mg, or 1 IU/mg and an anti-IIa activity less than about 40 IU/mg, 30 IU/mg, 20 IU/mg, 10 IU/mg, 5 IU/mg, 4 IU/mg, 3 IU/mg, 2 IU/mg, or 1 IU/mg); and (c) less than 50% glycol split uronic acid residues (e.g., less than 40%, 30%, 25%, or 20% glycol split uronic acid residues) in the preparation. In some instances, the preparation contains between 5% and 50% glycol split uronic acid residues (e.g., between 5% and 40%, 5% and 30%, 10% and 50%, 10% and 40%, or 10% and 30% glycol split uronic acid residues). In some instances, the preparation has an anti-Xa activity of less than 50 IU/mg, 40 IU/mg, 30 IU/mg or 20 IU/mg but greater than 0.5 IU/mg, 1 IU/mg or 2 IU/mg and/or an anti-IIa activity of less than 50 IU/mg, 40 IU/mg, 30 IU/mg or 20 IU/mg but greater than 0.5 IU/mg, 1 IU/mg or 2 IU/mg). In some instances, the preparation has a molecular weight distribution described herein.

In another instance, the polysaccharide preparation (e.g., a heparin-derived preparation) has the following characteristics: (a) a weight average chain molecular weight between 3,500 and 7,000 Da (e.g., 4,300 to 7,000 Da; 4,500 to 7,000 Da, 5,000 to 7,000 Da); (b) an anti-Xa activity and an anti-IIa activity each less than 50 IU/mg (e.g., an anti-Xa activity less than about 40 IU/mg, 30 IU/mg, 20 IU/mg, 15 IU/mg, 10 IU/mg, 5 IU/mg, 4 IU/mg, 3 IU/mg, 2 IU/mg or 1 IU/mg and an anti-IIa activity less than about 40 IU/mg, 30 IU/mg, 20 IU/mg, 10 IU/mg, 5 IU/mg, 4 IU/mg, 3 IU/mg, 2 IU/mg or 1 IU/mg); (c) at least one polysaccharide chain having a glycol split uronic acid residue (U_{G}); and (d) the polysaccharide chains of the preparation have no more than 3 glycol split uronic acid residues (U_{G}) per polysaccharide chain (e.g., each polysaccharide chain has no more than 2 or no more than 1 glycol split uronic acid residue (U_{G}) per polysaccharide chain). The preparation includes one or more chains having a glycol split uronic acid residue (U_{G}). In some instances, the preparation has an anti-Xa activity of less than 50 IU/mg, 40 IU/mg, 30 IU/mg or 20 IU/mg but greater than 0.5 IU/mg, 1 IU/mg or 2 IU/mg and/or an anti-IIa activity of less than 50 IU/mg, 40 IU/mg, 30 IU/mg or 20 IU/mg but greater than 0.5 IU/mg, 1 IU/mg or 2 IU/mg). In some instances, the preparation has a molecular weight distribution described herein.

In another instance, the polysaccharide preparation (e.g., a heparin-derived preparation) has the following characteristics: (a) a weight average chain molecular weight between 3,500 and 7,000 Da (e.g., 4,300 to 7,000 Da; 4,500 to 7,000 Da, 5,000 to 7,000 Da); (b) an anti-Xa activity and an anti-IIa activity each less than 50 IU/mg (e.g., an anti-Xa activity less than about 40 IU/mg, 30 IU/mg, 20 IU/mg, 15 IU/mg, 10 IU/mg, 5 IU/mg, 4 IU/mg, 3 IU/mg, 2 IU/mg, or 1 IU/mg and an anti-IIa activity less than about 40 IU/mg, 30 IU/mg, 20 IU/mg, 10 IU/mg, 5 IU/mg, 4 IU/mg, 3 IU/mg, 2 IU/mg, or 1 IU/mg); and (c) polysaccharide chains of the preparation have on average no more than 3 glycol split uronic acid residues (U_{G}) per polysaccharide chain (e.g., on average no more than 2.5, no more than 2, no more than 1.5, or no more than 1 glycol split uronic acid residues (U_{G}) per polysaccharide chain. In some instances, the preparation has an anti-Xa activity of less than 50 IU/mg, 40 IU/mg, 30 IU/mg or 20 IU/mg but greater than 0.5 IU/mg, 1 IU/mg or 2 IU/mg and/or an anti-IIa activity of less than 50 IU/mg, 40 IU/mg, 30 IU/mg or 20 IU/mg but greater than 0.5 IU/mg, 1 IU/mg or 2 IU/mg). In some instances, the preparation has a molecular weight distribution described herein.

In another instance, the polysaccharide preparation (e.g., a heparin-derived preparation) has the following characteristics: (a) a weight average chain molecular weight between 3,500 and 7,000 Da (e.g., 4,300 to 7,000 Da; 4,500 to 7,000 Da, 5,000 to 7,000 Da); (b) an anti-Xa activity and an anti-IIa activity each less than 50 IU/mg (e.g., an anti-Xa activity less than about 40 IU/mg, 30 IU/mg, 20 IU/mg, 15 IU/mg, 10 IU/mg, 5 IU/mg, 4 IU/mg, 3 IU/mg, 2 IU/mg, or 1 IU/mg and an anti-IIa activity less than about 40 IU/mg, 30 IU/mg, 20 IU/mg, 10 IU/mg, 5 IU/mg, 4 IU/mg, 3 IU/mg, 2 IU/mg, or 1 IU/mg); and (c) the preparation has greater than 40% U_{2S}H_{NS,6S} disaccharide residues (e.g., greater than 50%, 60%, 70%, or 80% U_{2S}H_{NS,6S} disaccharide residues). In some instances, the preparation has a degree of desulfation less than 40% (e.g., less than 30%, 20%, or 10%). In some instances, the preparation has an anti-Xa activity of less than 50 IU/mg, 40 IU/mg, 30 IU/mg or 20 IU/mg but greater than 0.5 IU/mg, 1 IU/mg or 2 IU/mg and/or an anti-IIa activity of less than 50 IU/mg, 40 IU/mg, 30 IU/mg or 20 IU/mg but greater than 0.5 IU/mg, 1 IU/mg or 2 IU/mg). In some instances, the preparation has a molecular weight distribution described herein.

In another instance, the polysaccharide preparation (e.g., a heparin-derived preparation) lacks substantial anticoagulant activity (e.g., has substantially no anticoagulant activity or residual anticoagulant activity), wherein the preparation includes polysaccharides that include Formula I:

[U_{w}-H_{x,y,z}]ₘ~[U_{G}-H_{x,y,z}]ₙ

wherein U indicates a uronic acid residue and H indicates a hexosamine residue;
m and n are integers such that
m = 4-16 (e.g., 4-8, 4-9, 4-10, 4-11,4-12, 4-13, 4-14, or 4-15), and
n = 1-4 (e.g., 1-2 or 1-3);
w = -2OS or -20H;
x = NS or NAc;
y = -3OS or -30H;
z = -6OS or -60H;
and 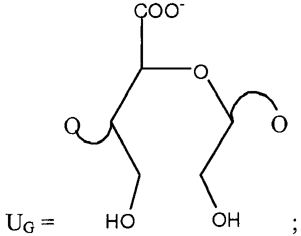
wherein the symbol ~ indicates that the units marked m and n are distributed along the polysaccharide chain and are not necessarily in sequence. For example, the following polysaccharide chain is encompassed by the above formula:

   [UG-H_{x,y,z}]-[U_{w}-H_{x,y,z}]-[U_{G}-H_{x,y,z}]-[U_{w}-H_{x,y,z}]-[U_{w}-H_{x,y,z}] - [U_{w}-H_{x,y,z}]

In addition, each of w, x, y, and z can be the same or different for each occurrence of [U_{w}-H_{x,y,z}], and each of x, y, and z can be the same or different for each occurrence of [U_{G}-Hₓ,_{y,z]}. Each occurrence of U can independently be an iduronic acid (I) or a glucuronic acid (G). In some instances, the preparation has an anti-Xa activity of less than 50 IU/mg, 40 IU/mg, 30 IU/mg or 20 IU/mg but greater than 0.5 IU/mg, 1 IU/mg or 2 IU/mg and/or an anti-IIa activity of less than 50 IU/mg, 40 IU/mg, 30 IU/mg or 20 IU/mg but greater than 0.5 IU/mg, 1 IU/mg or 2 IU/mg). In some instances, the preparation has a molecular weight distribution described herein.

In another instance, the polysaccharide preparation (e.g., a heparin-derived preparation) has a weight average chain molecular weight between 3,500 and 7,000 Da (e.g., 4,300 to 7,000 Da; 4,500 to 7,000 Da, 5,000 to 7,000 Da) and reduced anticoagulant activity, wherein the preparation includes polysaccharides that include Formula I:

[U_{w}-H_{x,y,z}]ₘ~[U_{G}-H_{x,y,z}]ₙ

wherein U indicates a uronic acid residue and H indicates a hexosamine residue;
m and n are integers such that
m = 4-16 (e.g., 4-8, 4-9, 4-10, 4-11, 4-12, 4-13, 4-14, or 4-15), and
n = 1-4 (e.g., 1-2 or 1-3);
w = -20S or -20H;
x = -NS or NAc;
y = -30S or -30H;
z = -6OS or -6OH;
and 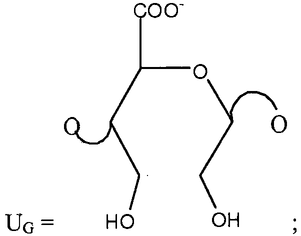
wherein the symbol ~ indicates that the units marked m and n are distributed along the polysaccharide chain and are not necessarily in sequence. For example, the following polysaccharide chain is encompassed by the above formula:

   [U_{G}-H_{x,y,z}]-[U_{w}-H_{x,y,z}]-[U_{G}-H_{x,y,z}]-[U_{w}-H_{x,y,z}]-[U_{w}-H_{x,y,z}]- [U_{w}-H_{x,y,z}]

In addition, each of w, x, y, and z can be the same or different for each occurrence of [U_{w}-H_{x,y},_{z]}, and each of x, y, and z can be the same or different for each occurrence of [U_{G}-H_{x,y,z}]. Each occurrence of U can independently be an iduronic acid (I) or a glucuronic acid (G). In some instances, the preparation has an anti-Xa activity of less than 50 IU/mg, 40 IU/mg, 30 IU/mg or 20 IU/mg but greater than 0.5 IU/mg, 1 IU/mg or 2 IU/mg and/or an anti-IIa activity of less than 50 IU/mg, 40 IU/mg, 30 IU/mg or 20 IU/mg but greater than 0.5 IU/mg, 1 IU/mg or 2 IU/mg). In some instances, the preparation has a molecular weight distribution described herein.

In another instance, the polysaccharide preparation (e.g., a heparin-derived preparation) lacks substantial anticoagulant activity (e.g., having substantially no anticoagulant activity or residual anticoagulant activity) and has antimetastatic activity, wherein the preparation includes polysaccharides that include Formula II:

[U_{w}H_{x,y,z}]ₘ-[U_{G}-H_{x,y,z}]ₙ₋[U_{w}-H_{x,y,z}]ₒ-[U_{G}-H_{x,y,z]p}-[U_{w}-H_{x,y,z}]_{q}

wherein U indicates a uronic acid residue and H indicates a hexosamine residue;
wherein m-r are integers such that:
   m = 0-10;
   n= 0- 3;
   o = 0-10;
   p = 0-3;
   q = 0-10;
   w = -2OS or -2OH;
   x = NS or NAc;
   y = -3OS or -3OH;
   z = -6OS or -6OH;
   and 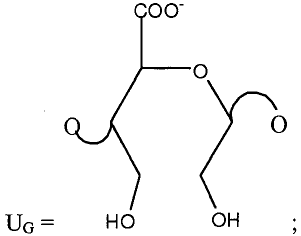
   wherein w, x, y, and z are each the same or different on each unit marked m, n, o, p, or q. In some instances, the sum of n + p is less than or equal to 4 (e.g., less than or equal to 3, 2, 1, or 0). In some instances, the sum of n and p is 4, 3, 2 or 1. In some instances, the sum of m, o and q is between 4 and 18, e.g., 4-8, 4-9, 4-10, 4-11, 4-12, 4-13, 4-14, 4-15, 4-16 or 4-17.

In addition, each of w, x, y, and z can be the same or different for each occurrence of [U_{w}-Hₓ,_{y,z}], and each of x, y, and z can be the same or different for each occurrence of [U_{G}-H_{x,y,z}]. Each occurrence of U can independently be an iduronic acid (I) or a glucuronic acid (G).

In some instances, the preparation has a weight average chain molecular weight between 3,500 and 8,000 Da, e.g., 4,300 and 7000 Da, 4,500 and 7,000 Da, 4,700 and 7,000 Da and 5,000 and 7,000 Da. In some instances, the preparation has an anti-Xa activity of less than 50 IU/mg, 40 IU/mg, 30 IU/mg or 20 IU/mg but greater than 0.5 IU/mg, 1 IU/mg or 2 IU/mg and/or an anti-IIa activity of less than 50 IU/mg, 40 IU/mg, 30 IU/mg or 20 IU/mg but greater than 0.5 IU/mg, 1 IU/mg or 2 IU/mg). In some instances, the preparation has a molecular weight distribution described herein.

In some instances, the preparation has a weight average chain molecular weight between 3,500 and 8,000 Da, e.g., 4,300 and 7000 Da, 4,500 and 7,000 Da, 4,700 and 7,000 Da and 5,000 and 7,000 Da.

Any of the preparations described herein, e.g., described above, can have other properties. E.g., one of the above described preparations or pharmaceutical compositions can further have one or more of the functional or structural properties set out below.

In one instance, at least one of the polysaccharide chains in the preparation has one of the following structures at the non-reducing end: 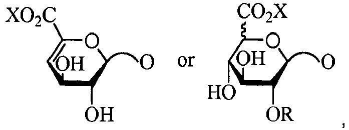 wherein X is H or Me and R is H or SO₃. For example, about 10%, 20%, 30%, 40%, 50%, 60%, 70%, 80%, 90%, or substantially all of the non-reducing ends of the preparation or pharmaceutical composition have the structure.

In one instance, at least one of the polysaccharide chains in the preparation or pharmaceutical composition includes a 2,5-anhydromannitol residue at the reducing end. For example, about 10%, 20%, 30%, 40%, 50%, 60%, 70%, 80%, 90%, or substantially all of the polysaccharide chains in the preparation or pharmaceutical composition include a 2,5-anhydromannitol residue at the reducing end.

In one instance, the preparation or pharmaceutical composition has a molecular weight distribution such that 10-50% (e.g., 10-40%, 10-30%, 15-30% or 15-25%) of the oligosaccharides of the preparation have a molecular weight < 3000 Da; 40-65% (e.g., 45-65%, 50-65%, or 55-65%) of the oligosaccharides have a molecular weight between 3000-8000 Da, and 5-30% (e.g., 10-30%, 15-30%, 10-25%, or 15-25%) of the oligosaccharides have a molecular weight > 8000 Da.

In one instance, the preparation has a polydispersity of about 1.2 to 1.7 (e.g., about 1.3 to 1.7, 1.2 to 1.6, or 1.3 to 1.6).

In one instance, the preparation or composition has anti-metastatic activity.

In one instance, the preparation or composition binds specifically to or inhibits an activity of one or more of: VEGF, FGF, HGF, HB-EGF, SDF-1-α, or P-selectin.

In one instance, the preparation or composition has a sodium content less than 30%, 25%, 20%, 15%, 10%. In one instance, the preparation or composition comprises: less than 20 ppm, 15 ppm, 10 ppm, 5 ppm iodine; less than 30%, 25%, 20%, 15%, 10% sulfur; less than 50, 40, 30, 20, 15 ppm boron.

The methods of making a preparation described herein include: combining UFH and nitrous acid (HONO) to produce a polysaccharide preparation; and, following nitrous acid treatment, performing reactions to produce a glycol split of at least a portion of the uronic acid residues in the preparation.

Methods of making a preparation include: depolymerizing an UFH (e.g., by chemical hydrolysis or enzymatic depolymerization); and, following depolymerization, performing reactions to produce a glycol split of at least a portion of the uronic acid residues in the preparation.

In one instance, reactions to produce a glycol split of at least a portion of the uronic residues in the preparation include oxidizing the polysaccharide preparation with periodate; and reducing the oxidized polysaccharide preparation with sodium borohydride. For example, the methods include oxidizing the polysaccharide preparation with periodate for about 10-20 hours at a temperature of about 0-10 °C; and following oxidation, reducing the sample with sodium borohydride for about 1 hour at a pH of about 5.0-8.0 at a temperature of about 0-10 °C.

Some methods of manufacturing a preparation described herein include: (1) depolymerizing an unfractionated heparin (UFH) (e.g., by nitrous acid depolymerization, hydrolytic depolymerization, or enzymatic depolymerization) to yield a polysaccharide preparation; (2) oxidizing the polysaccharide preparation with periodate; (3) reducing the oxidized polysaccharide preparation with sodium borohydride; and (4) isolating the polysaccharide preparation (e.g., by precipitating with a salt and a polar organic solvent, or by subjecting to a chromatographic separation or purification), to thereby make a preparation.

In one instance, the step of depolymerizing includes treating the UFH with about 0.01 to 0.05 M (e.g., about 0.02 to 0.04 M) nitrous acid at a pH of about 2 to 4 for about 1 to 5 hours at a temperature of about 10 to 30 °C .

In one instance, the step of oxidizing includes treating the polysaccharide preparation with about 0.05 to 0.2 M periodate for about 10 to 20 hours at a temperature of about 0 to 10 °C.

In one instance, the step of reducing comprises treating the oxidized polysaccharide preparation with about 0.5 to 2.0% (w/v) sodium borohydride for about 0.5 to 3 hours at a pH of about 6.0 to 7.0 and a temperature of about 0 to 10 °C.

In one instance, a method of making or manufacturing a polysaccharide preparation includes reducing the amount of boron in the preparation.

In one instance, the preparation is evaluated for a biological activity, e.g., anti-metastatic activity; binding to any of VEGF, FGF, HGF, HB-EGF, SDF-1α, and P-selectin; or inhibition of an activity of any of VEGF, FGF, HGF, HB-EGF, SDF-1α, and P-selectin.

A polysaccharide preparation that lacks substantial anticoagulant activity, as used herein, is one that has anti-Xa and anti-IIa activity each less than 100 IU/mg (e.g., less than 80 IU/mg, 70 IU/mg, or 60 IU/mg). In some instances, the polysaccharide preparation has substantially no anticoagulant activity, i.e., anti-Xa and anti-IIa activity each less than 50 IU/mg. In some instances, the polysaccharide preparation has anti-Xa and anti-IIa activity each less than 40, 30, 20, 25, 20, 15, 10, 5 IU/mg, 4 IU/mg, 3 IU/mg, 2IU/mg, 1 IU/mg or 0.5 IU/mg.

The degree of desulfation, as used herein, is defined as the percent reduction in moles of sulfate per moles of disaccharide unit as compared to unfractionated heparin.

The degree of sulfation, as used herein, is defined as the average number of moles of sulfate per moles of disaccharide unit.

Also disclosed herein is a method of treating a subject that includes administering a therapeutically effective amount of a polysaccharide preparation disclosed herein to the subject, and assessing the activity of the polysaccharide preparation as described herein. The terms "treating", "treatment", and the like, mean administering the preparation to a subject or a cell or tissue of a subject in order to obtain a desired pharmacological, physiological or clinical effect. Treatment with a polysaccharide preparation described herein may lessen, reduce, mitigate, ameliorate, delay, or prevent an existing unwanted condition or the onset or a symptom thereof. A "therapeutically effective amount" refers to an amount effective, at dosages and for periods of time necessary, to achieve the desired pharmacological, physiological or clinical effect in the subject.

The methods for treating a subject include methods for treating a subject having, or at risk of having, VEGF-, FGF-, SDF-1α- and/or selectin-mediated disease; an infectious disease, or a disease involving angiogenesis.

In one instance, the methods described herein are useful in methods for treating a subject having, or at risk of having, a metastatic disorder (e.g., a cancer, e.g., a carcinoma or other solid and hematological cancer). In those subjects, treatment may include, but is not limited to, inhibited tumor growth, reduction in tumor mass, reduction in size or number of metastatic lesions, inhibited development of new metastatic lesions, prolonged survival, prolonged progression-free survival, prolonged time to progression, and/or enhanced quality of life. In another instance, the subject may have a disorder or condition selected from the group consisting of: an inflammatory disorder, an autoimmune disease, a fibrotic or fibroproliferative disorder or an atopic disorder. Examples of inflammatory disorders include but are not limited to chronic obstructive pulmonary disease, asthma, rheumatoid arthritis, inflammatory bowel disease (including Crohn's disease and ulcerative colitis), multiple sclerosis, psoriasis, ischemia-reperfusion injuries, septic shock, age-related macular degeneration, atherosclerosis, Alzheimer's disease, cardiovascular disease, vasculitis, type I and II diabetes, metabolic syndrome, diabetic retinopathy, restenosis. Examples of autoimmune diseases include but are not limited to asthma, rheumatoid arthritis, inflammatory bowel disease, multiple sclerosis, psoriasis, type I diabetes, systemic lupus erythematosus (SLE), Sjögren's syndrome, Hashimoto's thyroiditis, Graves' disease, Guillain-Barré syndrome, autoimmune hepatitis, Myasthenia gravis. Examples of fibrotic diseases include but are not limited to scleroderma, chronic obstructive pulmonary disease, diabetic nephropathy, sarcoidosis, idiopathic pulmonary fibrosis, cirrhosis, cystic fibrosis, neurofibromatosis, endometriosis, post-operative fibroids, restenosis. Examples of atopic disease include but are not limited to atopic dermatitis, atopic asthma, and allergic rhinitis. The compositions of the present disclosure are administered to a subject having or at risk of developing one or more of the diseases in an effective amount for treating the disorder or condition.

In a preferred instance, the subject has, or is at risk of having, a cancer or metastatic disorder (e.g., a carcinoma). For example, the subject has a primary tumor and has, or is at risk of having, a metastasis of that primary tumor.

In one instance, the polysaccharide preparation is administered intravenously or subcutaneously or is inhaled.

In one instance, the polysaccharide preparation is administered in combination with another therapy, e.g., another therapeutic agent, e.g., a cytotoxic or cytostatic agent, and combinations thereof. In one instance, the polysaccharide preparation is administered in combination with surgery, radiotherapy, a chemotherapy agent, an antibody or a tyrosine kinase inhibitor. In one instance, the polysaccharide preparation is administered to a subject at a dose of 5-50 mg/kg.

In one instance, the polysaccharide preparation is administered chronically, e.g., at least twice over a specific period of time, e.g., at least twice during a period of six months. In one instance, a polysaccharide preparation is administered twice over a period of one week, two weeks, three weeks, one month, two months, three months, six months, one year, or even longer. The polysaccharide preparation can be administered daily (e.g., once, twice, or three or four times daily), once every other day, weekly (e.g., once, twice, or three times a week), once every other week, monthly, or any other chronic administration schedule.

For any of the ranges described herein, e.g., for a given structure or activity, the ranges can be those ranges disclosed as well as other ranges. For example, a range constructed from a lower endpoint of one range, e.g., for a given building block or activity, can be combined with the upper endpoint of another range, e.g., for the given building block or activity, to give a range.

An "isolated" or "purified" polysaccharide preparation is substantially free of cellular material or other contaminating proteins from the cell or tissue source from which the polysaccharide is derived, or substantially free from chemical precursors or other chemicals when chemically synthesized. "Substantially free" means that a preparation is at least 50% pure (wt/wt). In a preferred instance, the preparation has less than about 30%, 20%, 10% and more preferably 5% (by dry weight), of non-heparin-derived polysaccharides, proteins or chemical precursors or other chemicals, e.g., from manufacture. These are also referred to herein as "contaminants." Examples of contaminants that can be present in a polysaccharide preparation provided herein include, but are not limited to, sodium, sulfur, boron, enzyme (e.g., a heparinase enzyme), methanol, ethanol, iodine, and chloride.

Other features and advantages of the invention will be apparent from the following detailed description, and from the claims.

### BRIEF DESCRIPTION OF THE DRAWINGS

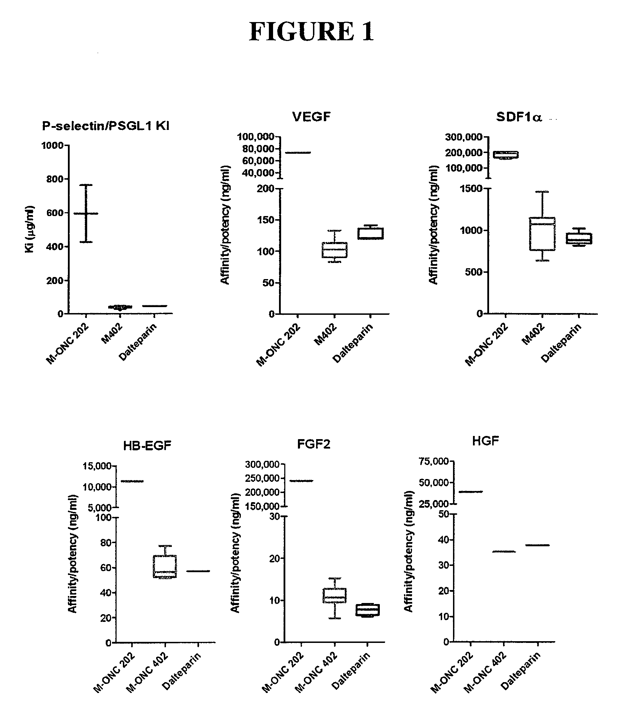

Figure 1 is a series of plots showing the binding affinities of M402, M-ONC 202 and Dalteparin to different heparin binding proteins as determined by Surface Plasmon Resonance.

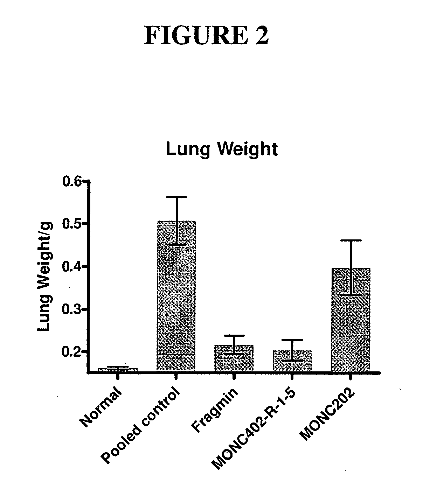

Figure 2 is a bar graph showing the effect of a polysaccharide preparation described herein in a murine melanoma experimental metastasis (B16F10 i.v.) model. Lung tumor burden (lung weight - normal lung weight) was determined for female C57BL/6 mice (9-10 weeks old) challenged with i.v. injection of 2x10⁵ B16F10 cells and pretreated with a single dose (10 mg/kg) of M402 (batch R-1-5), dalteparin (Fragmin®), or M-ONC 202 (negative control, N-desulfated polysaccharide) immediately before injection. "normal" designates unchallenged and untreated mice.

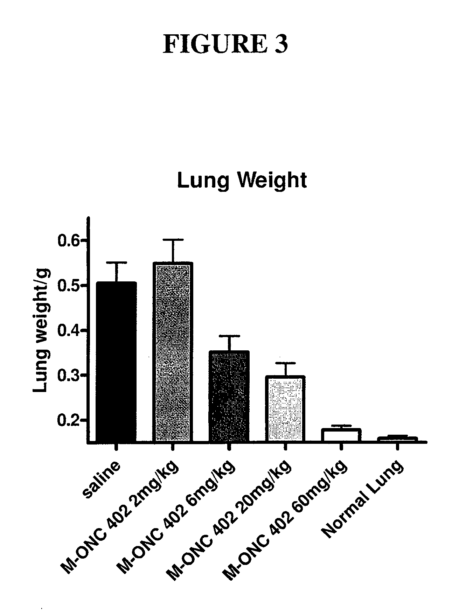

Figure 3 is a bar graph showing the dose response in groups of C57BL/6 mice (n=12 per group) treated with different doses of M402 (2mg/kg, 6mg/kg, 20mg/kg and 60mg/kg) followed by iv injection of 2x10⁵ B16F10 cells within 5 mins. The experiment was terminated on Day 21 and tumor colonization to the lung quantified by lung weight. M402 inhibited B16F10 tumor colonization to the lung in a dose-dependent manner.

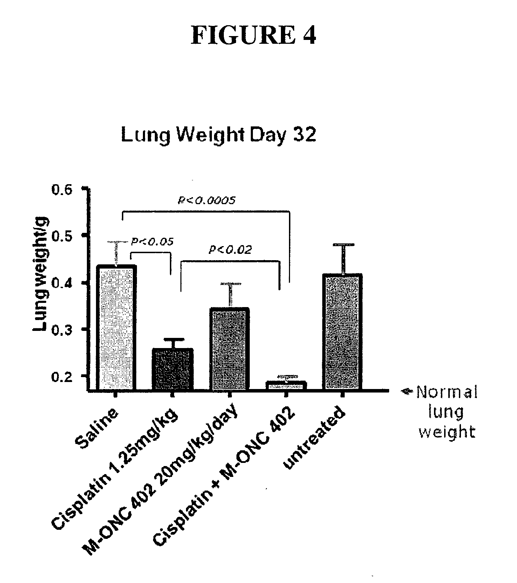

Figure 4 is a bar graph showing the effect of a polysaccharide preparation described herein in a 4T1 therapeutic model of breast cancer metastasis to the lung. Lung tumor burden (lung weight - normal lung weight) was determined on day 32 for female BALB/c mice (8 weeks old) challenged with intra-mammary fat pad injection of 8x10⁴ 4T1 cells and treated as indicated starting on day 5.

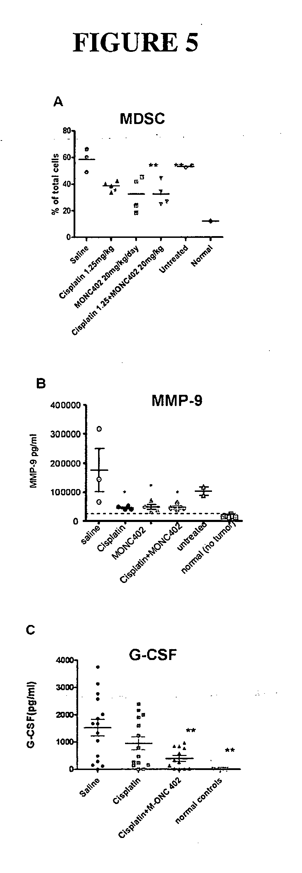

Figure 5 is a series of graphs showing the effect of a polysaccharide preparation described herein on: A) myeloid derived suppressor cells (MDSC); B) plasma MMP-9; and C) G-CSF levels, in 4T1 tumor-bearing mice.

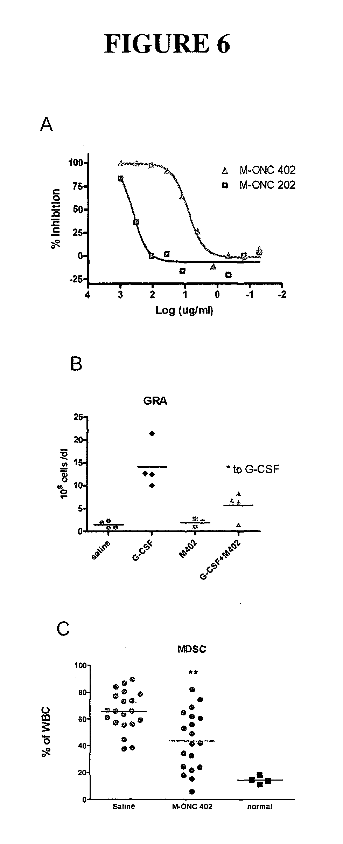

Figure 6 is a series of graphs showing the effect of a polysaccharide preparation described herein on stromal cell mobilization and recruitment. A) M402 inhibited SDF-1α-induced Jurkat cell migration in a dose-dependent manner. B) Flow cytometry of blood samples from mice injected with saline or G-CSF in the presence or absence of M402. C) M402 treatment significantly reduced MDSC in circulation in 4T1 tumor-bearing mice. *, P<0.05, **, P<0.01 by ANOVA.

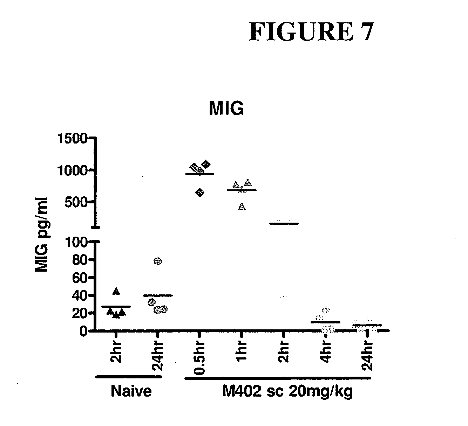

Figure 7 is a graph showing the effect of a polysaccharide preparation described herein on MIG expression levels in BALB/c mice were injected with saline or the polysaccharide preparation at 20mg/kg twice daily for 7 days.

### DETAILED DESCRIPTION

### Optimized Polysaccharide

In many clinical settings, commercially available LMWH preparations are preferred over UFH preparations as anticoagulants because LMWHs have more predictable pharmacokinetics and can be administered subcutaneously. However, because of the potential for bleeding complications due to their anticoagulant effects, currently available LMWH preparations are less suitable for therapy of non-coagulation mediated disorders, and/or for disorders that may require higher doses or chronic dosing regimens. The present disclosure features polysaccharide preparations designed to lack substantial anticoagulant activity while retaining clinically advantageous properties. Properties of the polysaccharide preparations include, e.g., lacking substantial anticoagulant activity, e.g., having substantially no anticoagulant activity (e.g., anti-IIa activity less than 50 IU/mg, anti-Xa activity less than 50 IU/mg), and having anti-metastatic, anti-angiogenic and/or anti-inflammatory activity.

Examples of such polysaccharide preparations include chains that include the following:

[U_{w}-H_{x,y,z}]ₘ~[U_{G}-H_{x,y,z}]ₙ

wherein U indicates a uronic acid residue and H indicates a hexosamine residue, wherein m and n are integers such that m = 6-18, and n = 1-4, w = -20S or -20H, x = -NS or -NAc, y = -30S or -30H, z = -6OS or -6OH,
and 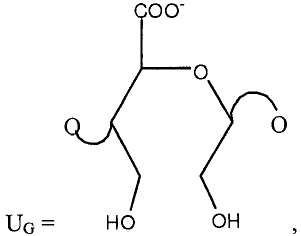 wherein the symbol ~ indicates that the units marked m and n are distributed along the polysaccharide chain and are not necessarily in sequence. For example, the following polysaccharide chain is encompassed by the above formula:

[U_{G}-H_{x,y,z}]-[U_{w}-H_{x,y,z}]-[U_{G}-H_{x,y,z}]-[U_{w}-H_{x,y,z}]-[U_{w}-H_{x,y,z}]- [U_{w}-H_{x,y,z}]

In addition, each ofw, x, y, and z can be the same or different for each occurrence of [U_{w}-H_{x,y,z]}, and each of x, y, and z can be the same or different for each occurrence of [U_{G}-H_{x,y,z]}. Each occurrence ofU can independently be an iduronic acid (I) or a glucuronic acid (G).

The polysaccharide preparation can have an anti-Xa activity and an anti-IIa activity each less than 50 IU/mg (e.g., an anti-Xa activity less than about 40 IU/mg, 30 IU/mg, 20 IU/mg, 15 IU/mg, 10 IU/mg, 5 IU/mg, 4 IU/mg, 3 IU/mg, 2 IU/mg or 1 IU/mg; or from about 0 to 50 IU/mg, about 0 to 40 IU/mg, about 0 to 30 IU/mg, about 0 to 25 IU/mg, about 0 to 20 IU/mg, about 0 to 10 IU/mg, about 0 to 5 IU/mg, about 5 to 10 IU/mg, about 5 to 15 IU/mg, or about 5 to 20 IU/mg; and an anti-IIa activity less than about 40 IU/mg, 30 IU/mg, 20 IU/mg, 15 IU/mg, 10 IU/mg, 5 IU/mg, 4 IU/mg, 3 IU/mg, 2 IU/mg or 1 IU/mg; or from about 0 to 50 IU/mg, about 0 to 40 IU/mg, about 0 to 30 IU/mg, about 0 to 25 IU/mg, about 0 to 20 IU/mg, about 0 to 10 IU/mg, about 0 to 5 IU/mg, about 5 to 10 IU/mg, about 5 to 15 IU/mg, or about 5 to 20 IU/mg); and

[U_{w}-H_{x,y,z}]ₘ-[U_{G}-H_{x,y,z}]ₙ-[U_{w}-Hₓ,_{y,z}]ₒ-[U_{G}-H_{x,y,z}]ₚ-[U_{w}-H_{x,y,z}]_{q}

wherein U indicates a uronic acid residue and H indicates a hexosamine residue, wherein m-r are integers such that: m = 0-10, n= 0- 3, o = 0-10, p = 0-3, q = 0-10, w = -2OS or -20H, x = NS or NAc, y = -30S or -30H, z = -6OS or -6OH,
and 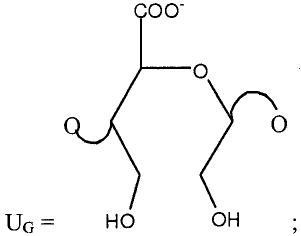 wherein w, x, y, and z are each the same or different on each unit marked m, n, o, p, or q. In some instances, the sum of n + p is less than or equal to 4 (e.g., less than or equal to 3, 2, 1, or 0). In some instances, the sum of n and p is 4, 3, 2 or 1. In some instances, the sum of m, o and q is between 4 and 18, e.g., 4-8, 4-9, 4-10, 4-11, 4-12, 4-13, 4-14, 4-15, 4-16 or 4-17.

In addition, each of w, x, y, and z can be the same or different for each occurrence of [U_{w}-H_{x,y,z}], and each of x, y, and z can be the same or different for each occurrence of [U_{G}-H_{x,y,z}]. Each occurrence of U can independently be an iduronic acid (I) or a glucuronic acid (G).

In some instances, the preparation has a weight average chain molecular weight between 3,500 and 7,000 Da, e.g., 4,300 and 7000 Da, 4,500 and 7,000 Da, 4,700 and 7,000 Da and 5,000 and 7,000 Da. The polysaccharide preparation can have an anti-Xa activity and an anti-IIa activity each less than 50 IU/mg (e.g., an anti-Xa activity less than about 40 IU/mg, 30 IU/mg, 20 IU/mg, 15 IU/mg, 10 IU/mg, 5 IU/mg, 4 IU/mg, 3 IU/mg, 2 IU/mg or 1 IU/mg; or from about 0 to 50 IU/mg, about 0 to 40 IU/mg, about 0 to 30 IU/mg, about 0 to 25 IU/mg, about 0 to 20 IU/mg, about 0 to 10 IU/mg, about 0 to 5 IU/mg, about 5 to 10 IU/mg, about 5 to 15 IU/mg, or about 5 to 20 IU/mg; and an anti-IIa activity less than about 40 IU/mg, 30 IU/mg, 20 IU/mg, 15 IU/mg, 10 IU/mg, 5 IU/mg, 4 IU/mg, 3 IU/mg, 2 IU/mg or 1 IU/mg; or from about 0 to 50 IU/mg, about 0 to 40 IU/mg, about 0 to 30 IU/mg, about 0 to 25 IU/mg, about 0 to 20 IU/mg, about 0 to 10 IU/mg, about 0 to 5 IU/mg, about 5 to 10 IU/mg, about 5 to 15 IU/mg, or about 5 to 20 IU/mg).

### Anti-IIa Activity

Polysaccharide preparations are disclosed herein that provide substantially reduced anti-IIa activity, e.g., anti-IIa activity of about, e.g., anti-Xa activity of about less than about 50 IU/mg, less than about 40 IU/mg, 30 IU/mg, 20 IU/mg, 15 IU/mg, 10 IU/mg, 5 IU/mg, 4 IU/mg, 3 IU/mg, 2 IU/mg or 1 IU/mg; or from about 0 to 50 IU/mg, about 0 to 40 IU/mg, about 0 to 30 IU/mg, about 0 to 25 IU/mg, about 0 to 20 IU/mg, about 0 to 10 IU/mg, about 0 to 5 IU/mg, about 5 to 10 IU/mg, about 5 to 15 IU/mg, or about 5 to 20 IU/mg). Anti-IIa activity is calculated in International Units of anti- IIa activity per milligram using statistical methods for parallel line assays. The anti-IIa activity levels described herein are measured using the following principle.

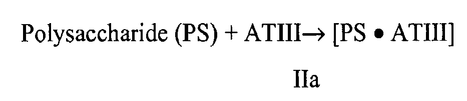

PS • ATIII→[PS • ATIII IIa] + IIa (Excess)

IIa (Excess) + Substrate → Peptide + pNA (measured spectrophotometrically)

Anti-factor IIa activity is determined by the sample potentiating effect on antithrombin (ATIII) in the inhibition of thrombin. Thrombin excess can be indirectly spectrophotometrically measured. The anti-factor IIa activity can be measured, e.g., on a Diagnostica Stago analyzer or on an ACL Futura3 Coagulation system, with reagents from Chromogenix (S-2238 substrate, Thrombin (53 nkat/vial), and Antithrombin), or on any equivalent system. Analyzer response is calibrated using the 2nd International Standard for Low Molecular Weight Heparin.

### Anti-Xa Activity

Preferably, a polysaccharide preparation provided herein has an anti-Xa activity of about 0 to 50 IU/mg, e.g., anti-Xa activity of about less than about 50 IU/mg, less than about 40 IU/mg, 30 IU/mg, 20 IU/mg, 15 IU/mg, 10 IU/mg, 5 IU/mg, 4 IU/mg, 3 IU/mg, 2 IU/mg or 1 IU/mg; or from about 0 to 50 IU/mg, about 0 to 40 IU/mg, about 0 to 30 IU/mg, about 0 to 25 IU/mg, about 0 to 20 IU/mg, about 0 to 10 IU/mg, about 0 to 5 IU/mg, about 5 to 10 IU/mg, about 5 to 15 IU/mg, or about 5 to 20 IU/mg). Anti-Xa activity of a preparation is calculated in International Units of anti-factor Xa activity per milligram using statistical methods for parallel line assays. The anti-factor Xa activity of preparations described herein is measured using the following principle:

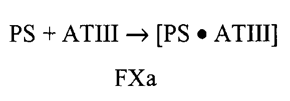

PS • ATIII → [PS • ATIII • FXa] + FXa(Excess)

FXa (Excess) + Substrate → Peptide + pNA (measured spectrophotometrically)

The anti-factor Xa activity is determined by the sample potentiating effect on antithrombin (ATIII) in the inhibition of activated Factor Xa (FXa). Factor Xa excess can be indirectly spectrophotometrically measured. Anti-factor Xa activity can be measured, e.g., on a Diagnostica Stago analyzer with the Stachrom® Heparin Test kit, on an ACL Futura3 Coagulation system with the Coatest® Heparin Kit from Chromogenix, or on any equivalent system. Analyzer response can be calibrated using the NIBSC International Standard for Low Molecular Weight Heparin.

### Molecular Weight and Chain Length

When weight average molecular weight of a preparation is determined, a weight average molecular weight of about 3500 to 8000 Da, about 3500 to 6300 Da, preferably about 4000 to 6000 Da, about 4200 to 5900, or about 4300 to 5800 Da, indicates that a significant number of chains in the polysaccharide preparation are of sufficient chain length.

"Weight average molecular weight" as used herein refers to the weight average in daltons of chains ofuronic acid/hexosamine disaccharide repeats. The presence of non-uronic acid and/or non-hexosamine building blocks are not included in determining the weight average molecular weight. Thus, the molecular weight of non-uronic acid and non-hexosamine building blocks within a chain or chains in the preparation should not be included in determining the weight average molecular weight. The weight average molecular weight (M_{w}) is calculated from the following equation: M_{w} = Σ(cᵢmᵢ)/ Σcᵢ. The variable cᵢ is the concentration of the polymer in slice i and mᵢ is the molecular weight of the polymer in slice *i*. The summations are taken over a chromatographic peak, which contains many slices of data. A slice of data can be pictured as a vertical line on a plot of chromatographic peak versus time. The elution peak can therefore be divided into many slices. The weight average molecular weight calculation is average dependant on the summation of all slices of the concentration and molecular weight. The weight average molar weight can be measured, e.g., using the Wyatt Astra software or any appropriate software. The weight average molecular weights described herein are determined by high liquid chromatography with two columns in series, for example a TSK G3000 SWXL and a G2000 SWXL, coupled with a multi angle light scattering (MALS) detector and a refractometric detector in series. The eluent used is a 0.2 M sodium sulfate, pH 5.0, and a flow rate of 0.5 mL/min.

A determination of whether a polysaccharide preparation includes chains of sufficient chain length can be made, for example, by determining the average chain length of the chains in the preparation and/or by determining the weight average molecular weight of chains within the preparation. When average chain length is determined, an average chain length of about 5 to 22, e.g., about 7 to 18, typically about 7 to 14 or 8 to 13 disaccharide repeats, indicates that a significant number of chains in the preparation are of sufficient chain length.

"Average chain length" as used herein refers to the average chain length of uronic acid/hexosamine disaccharide repeats that occur within a chain. The presence of non-uronic acid and/or non-hexosamine building blocks (e.g., attached PEG moieties) are not included in determining the average chain length. Average chain length is determined by dividing the number average molecular weight (Mn) by the number average molecular weight for a disaccharide (500 Da). Methods of determining number average molecular weight are described below using SEC MALS.

### Glycol Split Uronic Acids

A polysaccharide preparation described herein can include an opening of the glycoside ring, conventionally called reduction-oxidation (RO) derivatives. In these preparations, one or more glycoside rings having vicinyl diols that are opened, e.g., at the bond between C2 and C3, by means of an oxidation action, followed by a reduction. The compounds referred to herein will also be called "Glycol Split" derivatives.

In a further instance, the glycol split residues lend themselves to the subsequent functionalization. Therefore, the compounds may also bear equal or different groups, in place of the primary hydroxy groups deriving from glycol split, for example, aldehyde groups, methoxy groups, or oligosaccharide or peptide groups, ranging from a single saccharide or amino acid to more than one unit of length, e.g., 2 or 3 units.

In some instances, fewer than 50% of the uronic acid residues are glycol split uronic acid residues (e.g., less than 40%, 30%, 25%, or 20% of the uronic acid residues are glycol split uronic acid residues).

### Reducing End Structures

In some instances, at least about 50% of the chains in a polysaccharide preparation described herein have a modified reducing end structure such as a 2,5-anhydromannose residue or a 2,5-anhydromannose that has been reduced to form an alcohol. In some instances, at least about 55%, 60%, 65%, 70%, 75%, 80%, 85%, 90%, or 95% of the chains in the preparation have a modified reducing end structure, such that the reducing end includes a 2,5-anhydromannose residue or a 2,5-anhydromannose that has been reduced to form an alcohol.

### Polydispersity

The polydispersity of polysaccharide preparations provided herein is about 2 or less, e.g., 1.7 or less, e.g., about 1.7 or 1.6 to 1.2, about 1.4-1.5, and numbers in between.

The term "polydisperse" or "polydispersity" refers to the weight average molecular weight of a composition (Mw) divided by the number average molecular weight (Mn). The number average molecular weight (Mn) is calculated from the following equation: Mn = Σci/(Σci/mi). The variable ci is the concentration of the polysaccharide in slice i and Mi is the molecular weight of the polysaccharide in slice i. The summations are taken over a chromatographic peak, which contains many slices of data. A slice of data can be pictured as a vertical line on a plot of chromatographic peak versus time. The elution peak can therefore be divided into many slices. The number average molecular weight is a calculation dependent on the molecular weight and concentration at each slice of data. Methods of determining weight average molecular weight are described above, and were used to determine polydispersity as well.

### Methods of Making Polysaccharide Preparations

Various methods of making polysaccharide preparations, e.g., a preparation described herein, are also contemplated. One method includes providing a precursor heparin preparation having a weight average molecular weight of greater than 7000 Da or a chain length of greater than 7 to 18 disaccharides, and processing the precursor heparin preparation (e.g., by enzymatic or chemical depolymerization, e.g., by nitrous acid depolymerization) to obtain a polysaccharide preparation having a weight average molecular weight of about 3000 to 7000 Da or an average chain length of about 7 to 18 disaccharides. For example, the precursor heparin preparation can be unfractionated heparin.

The precursor heparin preparation can be processed by a method comprising depolymerization (e.g., by nitrous acid treatment, hydrolysis, or enzymatic depolymerization) followed by a glycol split reaction. Nitrous acid depolymerization can be accomplished, e.g., by treating the precursor heparin preparation (e.g., UFH) with nitrous acid (e.g., about 0.02 to 0.04 M nitrous acid) at a pH of about 2 to 4 for a specified period of time (e.g., about 1 to 5 hours) at a temperature of about 10 to 30 °C. The glycol split reaction involves periodate oxidation using periodate (e.g., about 0.05 M to 0.2 M sodium periodate) for about 10 to 20 hours at a temperature of about 0 to 10 °C. In some instances, residual impurities such as salts or diethylene glycol (DEG) can be subsequently removed by a chromatographic method, e.g. gel filtration chromatography. Optionally, the oxidized preparation is then reduced by treatment with a reducing agent (e.g., about 0.5 to 2.0% (w/v) sodium borohydride) for about 0.5 to 3 hours at a pH of about 6.0 to 7.0 and a temperature of about 0 to 10 °C.

A precursor heparin preparation can be processed using enzymatic digestion, chemical digestion or combinations thereof. Examples of chemical digestion include oxidative depolymerization, e.g., with H₂O₂ or Cu⁺ and H₂O₂, deaminative cleavage, e.g., with isoamyl nitrite or nitrous acid, β-eliminative cleavage, e.g., with benzyl ester, and/or by alkaline treatment. Enzymatic digestion can include the use of one or more heparin degrading enzymes. For example, the heparin degrading enzyme(s) can be, e.g., one or more heparinase, heparin lyase, heparin sulfate glycoaminoglycan (HSGAG) lyase, a lyase described as a glycoaminoglycan (GAG) lyase that can also degrade heparin. Preferably, the enzyme cleaves at one or more glycosidic linkages of unsulfated uronic acids.

### Biological Activities

The preparations described herein have anti-metastatic activity as assayed in an animal model of metastasis in which B16F10 melanoma cells injected into the tail veins of C57/BL mice arrest in the lungs and proliferate as discrete pulmonary foci. This assay is generally described in Gabri et al., 2006, Clin. Cancer Res., 12:7092-98. A preparation may additionally have activity in other experimental models of metastasis, including the C170HM2 assay, in which C170HM2 human colorectal cancer line cells are injected into the peritoneal cavity, where the primary site of metastasis is to the liver. The preparations described herein may also show anti-metastatic activity in spontaneous models of metastasis, such as the AP5LV model, in which AP5LV human colorectal cancer cells are implanted into the peritoneal wall and exhibit spontaneous metastasis to the lung, or the 4T1 model, in which 4T1 murine mammary carcinoma cells implanted in to the mammary fat pad exhibit spontaneous metastasis to the lung and other organs.

The preparations described herein can bind to and/or modulate (e.g., inhibit) an activity of one or more of VEGF, FGF, HGF, HB-EGF, SDF-1α, and P-selectin. In some instances, interaction of the preparation with (e.g., binding to) a target protein (e.g., VEGF, FGF, HGF, HB-EGF, SDF-1α, or P-selectin) can be assayed, e.g., in vitro, e.g., using methods known in the art. Numerous methods and techniques to detect binding or modulation (e.g., inhibition) of activity are known, e.g., standard receptor competition assays, fluorescence energy transfer (FET), fluorescence resonance energy transfer (FRET) (see, for example, U.S. Pat. No. 5,631,169; U.S. Pat. No. 4,868,103), and fluorescence polarization (FP). In some instances, evaluating binding of a polysaccharide preparation to a target protein can include a real-time monitoring of the binding interaction, e.g., using Biomolecular Interaction Analysis (BIA) (see, e.g., Sjolander and Urbaniczky (1991) Anal. Chem., 63:2338-2345 and Szabo et al. (1995) Curr. Opin. Struct. Biol., 5:699-705). Surface plasmon resonance or "BIA" detects biospecific interactions in real time, without labeling any of the interactants (e.g., BIAcore).

Activities of VEGF, FGF, HGF, HB-EGF, SDF-1α, and P-selectin on cells in vitro and in vivo are well known in the art. The ability of a polysaccharide preparation to modulate (e.g., inhibit) an activity of VEGF, FGF, HGF, HB-EGF, SDF-1α, or P-selectin can be assayed in vitro or in a cell-based assay or in vivo in an organism. For example, the ability of a polysaccharide preparation to modulate (e.g., inhibit) the activity of VEGF, FGF, HGF, HB-EGF, SDF-1α, or P-selectin to modulate (e.g., stimulate) the proliferation of endothelial cells, e.g., human umbilical vein epithelial cells, can be assayed. Exemplary methods of determining modulation of FGF activity can be found in U.S. Patent No. 5,733,893. A cell-based assay can be performed using a single cell, or a collection of at least two or more cells. The cell can be a yeast cell (e.g., *Saccharomyces cerevisiae*) or a mammalian cell, e.g., a cell line.

### Pharmaceutical Compositions

Compositions, e.g., pharmaceutically acceptable compositions, which include a preparation described herein, formulated together with a pharmaceutically acceptable carrier, are provided.

As used herein, "pharmaceutically acceptable carrier" includes any and all solvents, dispersion media, isotonic and absorption delaying agents, and the like that are physiologically compatible with parenteral administration. The carrier can be suitable for any parenteral administration, e.g., intravenous, intramuscular, subcutaneous, intraocular, rectal, inhaled or spinal administration (e.g., by injection or infusion).

The compositions of this disclosure may be in a variety of forms. These include, for example, liquid, semi-solid and solid dosage forms, such as liquid solutions (e.g., injectable and infusible solutions), dispersions or suspensions, and liposomes. The preferred form depends on the intended mode of administration and therapeutic application. Typical preferred compositions are in the form of injectable or infusible solutions. The preferred mode of administration is parenteral (e.g., intravenous, subcutaneous, intraocular, intraperitoneal, intramuscular). In a preferred instance, the preparation is administered by intravenous infusion or injection. In another preferred instance, the preparation is administered by intramuscular or subcutaneous injection.

The phrases "parenteral administration" and "administered parenterally" as used herein means modes of administration other than enteral and topical administration, usually by injection, and includes, without limitation, intravenous, intramuscular, subcutaneous, intraarterial, intrathecal, intracapsular, intraorbital, intravitreous, intracardiac, intradermal, intraperitoneal, transtracheal, inhaled, subcutaneous, subcuticular, intraarticular, subcapsular, subarachnoid, intraspinal, epidural and intrasternal injection and infusion.

Therapeutic compositions typically should be sterile and stable under the conditions of manufacture and storage. The composition can be formulated as a solution, microemulsion, dispersion, liposome, or other ordered structure suitable to high concentration. Sterile injectable solutions can be prepared by incorporating the active compound (i.e., polysaccharide preparation) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, various polymers, monostearate salts and gelatin.

For many therapeutic applications, the preferred route/mode of administration is intravenous injection or infusion. As will be appreciated by the skilled artisan, the route and/or mode of administration will vary depending upon the desired results.

Formulations for injection may be presented in unit dosage form, e.g., in ampoules, syringes, syringe pens, or in multi-dose containers, e.g., with an added preservative. The compositions may take such forms as suspensions, solutions or emulsions in oily or aqueous vehicles, and may contain formulatory agents such as suspending, stabilizing and/or dispersing agents.

For administration by inhalation, the preparation may be conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. Capsules and cartridges of, e.g., gelatin for use in an inhaler or insufflator may be formulated containing a powder mix of the compound and a suitable powder base such as lactose or starch. In addition, dry powder formations for inhalation therapy are within the scope of the invention. Such dry powder formulations may be prepared as disclosed, e.g., in WO 02/32406.

In addition to the compositions described previously, the compounds may also be formulated as a depot preparation. Such long-acting formulations may be formulated with suitable polymeric or hydrophobic materials (for example, as an emulsion in an acceptable oil) or ion exchange resins, or as sparingly soluble derivatives, for example, as a sparingly soluble salt.

The pharmaceutical compositions also may comprise suitable solid or gel phase carriers or excipients. The compositions can be included in a container, pack, or dispenser together with instructions for administration.

The preparation can also be administered with short or long term implantation devices, e.g., a stent. The preparation can be implanted subcutaneously, can be implanted into tissues or organs (e.g., the coronary artery, carotid artery, renal artery and other peripheral arteries, veins, kidney, heart cornea, vitreous, cerebrum, etc.), or can be implanted in physiological spaces around tissues and organs (e.g., kidney capsule, pericardium, thoracic or peritoneal space).

The preparation can also be used to coat various medical devices. For example, the preparation can be used to coat a stent or extracorporeal circuit. Such formulations of the preparations may include using, e.g., controlled release beads, gel or microspheres as well as various polymers such as PLGA, cellulose, alginate or other polysaccharides.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms are dictated by and directly dependent on (a) the unique characteristics of the active compound and the particular therapeutic effect to be achieved, and (b) the limitations inherent in the art of compounding such an active compound for the treatment of sensitivity in individuals.

It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions.

The pharmaceutical compositions of the present disclosure may include a therapeutically effective amount of a preparation. A therapeutically effective amount of the preparation may vary according to factors such as the disease state, age, sex, and weight of the individual and can include more than one unit dose. A therapeutically effective amount is also one in which any toxic or detrimental effects of the preparation are outweighed by the therapeutically beneficial effects. A therapeutically effective amount may inhibit a measurable parameter, e.g., VEGF activity, FGF activity, HGF activity, HB-EGF activity, SDF-1α activity, P-selectin activity, or size or rate of growth of metastatic lesions, e.g., by at least about 20%, more preferably by at least about 25%, 30%, 40%, even more preferably by at least about 50%, 60%, and still more preferably by at least about 70%, 80% relative to untreated subjects. The ability of a compound to inhibit a measurable parameter, e.g., metastasis or angiogenesis, can be evaluated in an animal model system or in a human (e.g., in a pre-clinical model or a clinical trial). Alternatively, a property of a composition can be evaluated by examining the activity of the compound in an *in vitro* assay. Exemplary doses for intravenous or subcutaneous administration of the polysaccharide preparation are about 0.03 mg/kg to 0.45 mg/kg, e.g., 0.03 mg/kg, 0.05 mg/kg, 0.1 mg/kg, 0.15 mg/kg, 0.2 mg/kg, 0.22 mg/kg, 0.25 mg/kg, 0.27 mg/kg, 0.3 mg/kg, 0.35 mg/kg, 0.37 mg/kg, 0.4 mg/kg, 0.44 mg/kg, preferably about 0.1 mg/kg, 0.15 mg/kg, 0.2 mg/kg, 0.25 mg/kg, 0.3mg/kg, 0.35 mg/kg, 0.4 mg/kg, 0.44 mg/kg, 0.47 mg/kg, 0.5 mg/kg, 0.55 mg/kg, 0.60 mg/kg, 0.7 mg/kg, preferably about 0.30 to 0.50 mg/kg, e.g., 0.30mg/kg, 0.35mg/kg, 0.40 mg/kg, 0.42 mg/kg, 0.44 mg/kg, 0.47 mg/kg or 0.50 mg/kg. In some instances, the polysaccharide preparation can be administered at a dose between 1-80 mg/kg, between 1-40 mg/kg, between 1-30 mg/kg, e.g., between 5-50 mg/kg/day.

### Detection of Biomarkers

The polysaccharide preparations described herein may be evaluated by measuring the level of one or more biomarkers, e.g., after administration of a polysaccharide preparation to a subject. Biomarkers (also called "biological markers") are biological characteristics that can be objectively measured and evaluated as indicators of biological processes, e.g., as indicators of a pathogenic process, and/or of a response to a therapeutic intervention.

A biomarker is virtually any biological compound, such as a specific type of cell, a protein or a fragment thereof, a peptide, a polypeptide, a proteoglycan, a glycoprotein, a lipoprotein, a carbohydrate, a lipid, a nucleic acid, an organic on inorganic chemical, a natural polymer, or a small molecule, that is present in the biological sample and that may be isolated from, or measured in, the sample. Biological markers can reflect a variety of disease characteristics, including the level of exposure to an environmental or genetic trigger, an element of the disease process itself, an intermediate stage between exposure and disease onset, an independent factor associated with the disease state but not causative of pathogenesis. As such, a biomarker can be indicative of the activity (e.g., of the efficacy) of a therapeutic intervention.

A variety of methods can be used to determine the level of a biomarker of interest, e.g., a biomarker described herein. In general, these methods include contacting a sample (in vitro or in vivo) with a probe for the biomarker. In some cases, the probe is a detector, such as a detector for a type of cell (e.g., FACS analysis). In some cases, the probe is an agent that selectively binds to the biomarker, such as an antibody, with a sample to evaluate the level of biomarker in the sample. In one instance, the antibody includes a detectable label. Antibodies can be polyclonal or monoclonal. An intact antibody, or a fragment thereof (e.g., Fab or F(ab')₂) can be used. The term "labeled," with regard to the probe or antibody, is intended to encompass direct labeling of the probe or antibody by coupling (i.e., physically linking) a detectable substance to the probe or antibody, as well as indirect labeling of the probe or antibody by reactivity with a detectable substance.

The detection methods can be used to detect a biomarker in a biological sample *in vitro* as well as *in vivo*. *In vitro* techniques for detection of biomarkers include enzyme linked immunosorbent assays (ELISAs), immunoprecipitations, immunofluorescence, enzyme immunoassays (EIA), radioimmunoassays (RIA), Western blot analysis and flow cytometry. *In vivo* techniques for detection of biomarkers include introducing into a subject a labeled antibody that is specific for the biomarker of interest. For example, the antibody can be labeled with a radioactive marker, e.g., a radioisotope, whose presence and location in a subject can be detected by standard imaging techniques.

In some instances, tumor stromal levels of a biomarker of interest may be measured, e.g., by measuring in a tumor lysate or a tumor culture.

### Uses

The polysaccharide preparations can be used to treat a subject. As used herein, a subject is a mammal, e.g., a non-human experimental mammal, a veterinary mammal, or a human. Non-human mammals include a primate, cow, horse, pig, sheep, goat, dog, cat, or rodent.

The preparations provided herein can be used, for example, to treat or prevent a metastatic disorder (e.g., a cancer, e.g., a carcinoma or other solid or hematological cancer). As used herein, the term "cancer" is meant to include all types of cancerous growths or oncogenic processes, metastatic tissues or malignantly transformed cells, tissues, or organs, irrespective of histopathologic type or stage of invasiveness. Methods and compositions disclosed herein are particularly useful for treating, or reducing the size, numbers, or rate of growth of, metastatic lesions associated with cancer.

Examples of cancers include, but are not limited to, solid tumors, soft tissue tumors, hematopoietic tumors and metastatic lesions. Examples of solid tumors include malignancies, e.g., sarcomas, adenocarcinomas, and carcinomas, of the various organ systems, such as those affecting head and neck (including pharynx), thyroid, lung (small cell or non small cell lung carcinoma), breast, lymphoid, gastrointestinal (e.g., oral, esophageal, stomach, liver, pancreas, small intestine, colon and rectum, anal canal), genitals and genitourinary tract (e.g., renal, urothelial, bladder, ovarian, uterine, cervical, endometrial, prostate, testicular), CNS (e.g., neural or glial cells, e.g., neorublastoma or glioma), skin (e.g., melanoma). Examples of hematopoietic cancers that can be treated include multiple myeloma, lymphomas and leukemias and myelodysplasia. Methods and compositions disclosed herein are particularly useful for treating, e.g., reducing or delaying, metastatic lesions associated with the aforementioned cancers. In some instances, the patient will have undergone one or more of surgical removal of a tissue, chemotherapy, or other anti-cancer therapy and the primary or sole target will be metastatic lesions, e.g., metastases in the bone or lymph nodes or lung or liver or peritoneal cavity or the CNS or other organs.

The methods of the present disclosure, e.g., methods of treatment, can further include the step of monitoring the subject, e.g., for a change (e.g., an increase or decrease) in one or more of: tumor size; levels of a cancer marker, for a patient with cancer; the size or rate of appearance of new lesions, e.g., in a scan; the appearance of new disease-related symptoms; the size of soft tissue mass, e.g., a decrease or stabilization; quality of life, e.g., amount of disease associated pain, e.g., bone pain; or any other parameter related to clinical outcome. The subject can be monitored in one or more of the following periods: prior to beginning of treatment; during the treatment; or after one or more elements of the treatment have been administered. Monitoring can be used to evaluate the need for further treatment with the same preparation or for additional treatment with additional agents. Generally, a decrease in one or more of the parameters described above is indicative of the improved condition of the subject.

The preparations described herein can be administered to a subject in single or multiple doses to treat or prevent a metastatic or cancerous disorder, e.g., a cancerous disorder described herein.

The preparations described herein can also be used to treat inflammatory, autoimmune, fibrotic, fibroproliferative, atopic, or angiogenic disorders. Examples of inflammatory disorders include but are not limited to chronic obstructive pulmonary disease, asthma, rheumatoid arthritis, inflammatory bowel disease (including Crohn's disease and ulcerative colitis), multiple sclerosis, psoriasis, ischemia-reperfusion injuries, septic shock, age-related macular degeneration, atherosclerosis, Alzheimer's disease, Parkinson's disease, cardiovascular disease, vasculitis, type I and II diabetes, metabolic syndrome, diabetic retinopathy, restenosis. Examples of autoimmune diseases include but are not limited to asthma, rheumatoid arthritis, inflammatory bowel disease, multiple sclerosis, psoriasis, type I diabetes, systemic lupus erythematosus (SLE), Sjögren's syndrome, Hashimoto's thyroiditis, Graves' disease, Guillain-Barré syndrome, autoimmune hepatitis, Myasthenia gravis. Examples of fibrotic diseases include but are not limited to scleroderma, chronic obstructive pulmonary disease, diabetic nephropathy, sarcoidosis, idiopathic pulmonary fibrosis, cirrhosis, cystic fibrosis, neurofibromatosis, endometriosis, post-operative fibroids, restenosis. Examples of atopic disease include but are not limited to atopic dermatitis, atopic asthma, and allergic rhinitis.

Examples of fibroproliferative disorders include systemic and local scleroderma, keloids and hypertrophic scars, atherosclerosis, restenosis, fibrosarcoma, neurofibromatosis, and rheumatoid arthritis. Examples of scarring associated with trauma include scarring due to surgery, chemotherapeutic-induced fibrosis, radiation-induced fibrosis, scarring associated with injury or burns.

In one instance, the polysaccharide preparations are used for inhibiting angiogenesis, e.g., to treat angiogenic disorders. Angiogenesis as used herein is the inappropriate formation of new blood vessels. Angiogenic disorders include, but are not limited to, tumors, neovascular disorders of the eye, endometriosis, macular degeneration, osteoporosis, psoriasis, arthritis, cancer and cardiovascular disorders. It is understood that some disorders will fall within more than one category of disease described herein.

The preparations described herein can also be used to treat or prevent infectious disorders such as, e.g., malaria.

### Combination Therapy

The methods and compositions of the present disclosure can be used in combination with other therapeutic modalities. Administered "in combination", as used herein, means that two (or more) different treatments are delivered to the subject during the course of the subject's affliction with the disorder, such that the effects of the treatments on the patient overlap at a point in time. In some instances, the delivery of one treatment is still occurring when the delivery of the second begins, so that there is overlap in terms of administration. This is sometimes referred to herein as "simultaneous" or "concurrent delivery." In other instances, the delivery of one treatment ends before the delivery of the other treatment begins. In some instances of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some instances, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered.

In one instance, the methods include administering to the subject a preparation described herein, in combination with one or more additional therapies, e.g., surgery, radiation therapy, or administration of another therapeutic preparation. In one instance, the additional therapy may include chemotherapy, e.g., a cytotoxic agent. In one instance the additional therapy may include a targeted therapy, e.g. a tyrosine kinase inhibitor, a proteasome inhibitor, a protease inhibitor. In one instance, the additional therapy may include an anti-inflammatory, anti-angiogenic, anti-fibrotic, or anti-proliferative compound, e.g., a steroid, a biologic immunomodulator, a monoclonal antibody, an antibody fragment, an aptamer, an siRNA, an antisense molecule, a fusion protein, a cytokine, a cytokine receptor, a bronchodialator, a statin, an anti-inflammatory agent (e.g. methotrexate), an NSAID. In another instance, the additional therapy could include combining therapeutics of different classes. The polysaccharide preparation and the additional therapy can be administered simultaneously or sequentially.

Exemplary cytotoxic agents that can be administered in combination with the polysaccharide preparation include antimicrotubule agents, topoisomerase inhibitors, antimetabolites, protein synthesis and degradation inhibitors, mitotic inhibitors, alkylating agents, platinating agents, inhibitors of nucleic acid synthesis, histone deacetylase and DNA methyltransferase inhibitors, nitrogen mustards, nitrosoureas, ethylenimines, alkyl sulfonates, triazenes, folate analogs, nucleoside analogs, ribonucleotide reductase inhibitors, vinca alkaloids, taxanes, epothilones, intercalating agents, agents capable of interfering with a signal transduction pathway, agents that promote apoptosis and radiation, antibody conjugates that bind surface proteins to deliver a toxic agent. In one instance, the cytotoxic agent that can be administered with a preparation described herein is platinum, cyclophosphamide, dacarbazine" methotrexate, fluorouracil, gemcitabine, capecitabine, hydroxyurea, topotecan, irinotecan, azacytidine, vorinostat, ixabepilone, bortezomib, taxanes (paclitaxel, docetaxel), cytochalasin B, gramicidin D, ethidium bromide, emetine, mitomycin, etoposide, tenoposide, vincristine, vinblastine, vinorelbine, colchicin, anthracyclines (doxorubicin and epirubicin) daunorubicin, dihydroxy anthracin dione, mitoxantrone, mithramycin, actinomycin D, adriamycin, 1-dehydrotestosterone, glucocorticoids, procaine, tetracaine, lidocaine, propranolol, puromycin, ricin, and maytansinoids.

The combination therapy can also include a composition of the present disclosure coformulated with, and/or coadministered with, one or more additional therapeutic agents, e.g., one or more anti-cancer agents, cytotoxic or cytostatic agents, hormone treatment, small molecule inhibitors of receptor tyrosine kinases and other tyrosine kinases including HER-2, EGFR, VEGFR, BCR-ABL, c-KIT (such as Gefitinib, Erlotinib, Lapatinib, Sorafenib, Sunitinib, Imatinib, Dasatinib, Nilotinib) or mTOR (such as temsirolimus, everolimus, rapamycin), or cytokines or chemokines, vaccines, antibodies against cell membrane receptors pathways including EGF-EGFR, VEGF-VEGFR, CD 19, CD20, CD3, CTLA-4 (such as Trastuzumab, Cetuximab, Panitumumab, Bevacizumab, Rituximab, Tositumomab) and/or other immunotherapies.

### Other Embodiments

This invention is further illustrated by the following examples that should not be construed as limiting.

### EXAMPLES

### Example 1: Preparation of a Polysaccharide Preparation

This example describes the production of a polysaccharide preparation described herein.

**Overview:** Glycol Split low molecular weight heparin alcohol (GS-LMWH-CH₂-OH) was generated from unfractionated heparin (UFH) by controlled nitrous acid depolymerization followed by oxidative glycol-splitting and subsequent reduction to an alcohol. In the first step, UFH was depolymerized to obtain depolymerized heparin (DPH-CHO) having an anhydromannose moiety at the reducing end of the polysaccharide. This was followed by Step II oxidative cleavage of the 2, 3-diols present in the depolymerized heparin with sodium periodate to generate ring opened glycol split residues along the heparin chain (GS-DPH-CHO). The Step III involved a reduction step, wherein the aldehydic moieties are converted to alcohols using sodium borohydride to generate Glycol Split low molecular weight heparin alcohol.

### Method Overview:

The following paragraphs describe the preparation and properties of a polysaccharide preparation described herein.

### 1. Depolymerization:

Unfractionated Heparin (10 g) was dissolved in 100 mL of de-ionized water equilibrated at room temperature. The pH of this solution was subsequently lowered to pH 3.1, following which sodium nitrite (0.03 M) was added. This reaction solution was allowed to stir for 3 hours following which the pH was neutralized prior to addition of sodium chloride (10 g). After complete dissolution of salt, methanol (200 mL) was added to this solution with constant stirring. The precipitate obtained was then aged at 6 °C for 2 hours. This precipitate was then filtered and dried to obtain DPH in 80-85% yield and possessing the following characteristics:

| | |
|---|---|
| Mw: | 5300-6100 |
| Mw Distribution: | (i) <3000 Daltons: 23-30% |
| | (ii) 3000-8000 Daltons: 50-55% |
| | (iii) >8000 Daltons : 15-22% |
| Anti-Xa Activity: | 80-120 IU/mg |
| Anti-IIa Activity: | 40-70 IU/mg |

### 2. Periodate Oxidation

The aldehyde (5 g) obtained in Step I was dissolved in 50 mL water equilibrated at 5 °C. To this solution was added cooled NaIO₄ solution (0.1 M, 50 mL) and the reaction mixture was allowed to stir in the absence of light for 16 hours. On completion, the reaction was quenched by the addition of diethylene glycol (10 mL), following which the temperature was raised back to room temperature. Five grams of sodium chloride was then added to this solution, followed by addition of 150 mL methanol to precipitate the heparin. The precipitate was allowed to age at 6 °C for 2 hours before filtration and drying to yield a glycol-split polysaccharide (95-98% yield) with the following characteristics:

| | |
|---|---|
| Mw: | 5000-5800 |
| Mw Distribution: | (i) <3000 Daltons: 25-30% |
| | (ii) 3000-8000 Daltons: 55-60% |
| | (iii) >8000 Daltons: 15-20% |

### 3. Reduction

The glycol split polysaccharide (4 g) obtained above in Step II was dissolved in 40 mL water maintained at 5 °C. To this solution was added sodium borohydride (0.4 g) and the reaction mixture subsequently stirred for 1 hour. After 1 hour, the reaction mixture was brought to room temperature, followed by the addition of sodium chloride (4 g). Following salt dissolution, methanol (80 mL) was added to this solution accompanied with constant stirring. The precipitate thus obtained was then allowed to age at 6 °C for 2 hours before filtration and drying to yield the desired product. A polysaccharide preparation with the following characteristics was thus obtained in 55-60% yield:

| | |
|---|---|
| Mw: | 5500-6200 |
| Mw Distribution: | (i) <3000 Daltons: 17-23% |
| | (ii) 3000-8000 Daltons: 56-62% |
| | (iii) >8000 Daltons: 17-22% |
| Anti-Xa Activity: | 5-20 IU/mg |
| Anti-IIa Activity: | 1-10 IU/mg |

### Example 2: Anti-metastatic Properties of Polysaccharide Preparations

This example shows that the polysaccharide preparations have anti-cancer and anti-metastatic activity in multiple models of metastasis.

### Model A: Murine melanoma experimental metastasis (B16F10 iv) model

A polysaccharide preparation produced as described in Example 1 (herein referred to as "M402") showed anti-metastasis activity in a murine melanoma experimental metastasis model.

Female C57BL/6 mice (9-10 weeks old) were treated once with a single dose (10 mg/kg) of M402, dalteparin/Fragmin® (a LMWH which has been reported to decrease metastasis), or M-ONC 202 (negative control, N-desulfated polysaccharide) immediately before i.v. injection of 2x10⁵ B16F10 cells. Mice were sacrificed on day 21 and tumor burden was calculated as lung weight-normal lung weight. As shown in Figure 1, M402 significantly inhibited B16F10 colonization of the lung relative to a pooled (untreated) control.

### Model B: Colon cancer metastasis to the liver

M402 showed prophylactic anti-metastasis activity in an orthotopic liver metastasis model.

Liver metastasis was initiated by intraperitoneal injection of C170HM2 human colorectal tumor cells into male MF1 nude (nu/nu) athymic mice. 5FU/leucovorin was used as a positive control.

C170HM2 cells were maintained in vitro in RPMI culture medium (Sigma) containing 10% (v/v) heat inactivated fetal bovine serum and 2 mM L-glutamine at 37 °C in 5% CO₂ and humidified conditions. Cells from sub-confluent monolayers were harvested with 0.025% EDTA, washed in culture medium and re-suspended in sterile phosphate buffered saline, pH 7.4 (PBS) for in vivo administration. 1.5 × 10⁶ cells in a volume of 1 ml were injected intraperitoneally into 65 mice, and the mice were allocated into treatment groups as below.

| | |
|---|---|
| Group 1: n = 10 | Vehicle control |
| Group 2: n = 10 | 25 mg/kg 5FU/leucovorin i.v. cycled on days 1, 3, 5, 7 |
| Group 3: n = 10 | 5 mg/kg compound 1 (Dalteparin) s.c. once daily |
| Group 4: n = 10 | 5 mg/kg compound 2 (M402) s.c. once daily |
| Group 5: n = 10 | 15 mg/kg compound 2 s.c. once daily |
| Group 6: n = 10 | 30 mg/kg compound 2 s.c. once daily |
| Group 7: n = 5 | Untreated |

Treatment was initiated on day 1 following cell injection and continued until day 35 or until the clinical condition of the animal required termination. Groups 5 and 6 missed one dose on day 5. No adverse affects of the test compounds in mice bearing the tumors were observed.

The study was terminated on day 35, and the tumors in the liver were excised and weighed. The numbers of lung nodules are also counted. The mean liver tumor weights and cross-sectional area are summarized in Table 1.

**Table 1. C170HM2 model: summary of mean liver tumor weight and statistical analysis**

| Group | Treatment | Mean tumor weight | | | Mean tumor area | | |
|---|---|---|---|---|---|---|---|
| | | (g) | (% of vehicle) | One way ANOVA | (mm²) | (% of vehicle) | One way ANOVA |
| 1 | Vehicle | 0.097 | 100.00 | - | 34.18 | 100.00 | - |
| 2 | 5FU/Leu | 0.037 | 11.94 | p = 0.006 | 13.12 | 15.7 | p = 0.011 |
| 3 | 5 mg/kg Dalteparin | 0.018 | 18.56 | p = 0.017 | 8.09 | 23/67 | p = 0.031 |
| 4 | 5 mg/kg M402 | 0.057 | 58.76 | NS | 18.34 | 53.66 | NS |
| 5 | 15 mg/kg M402 | 0.010 | 10.31 | p = 0.007 | 6.95 | 20.33 | p = 0.016 |
| 6 | 30 mg/kg M402 | 0.003 | 3.09 | p = 0.004 | 0.96 | 2.80 | p = 0.004 |
| 7 | Untreated control | 0.31 | - | p = 0.035 | 83.58 | 244.53 | p = 0.084 |

| | | | | | | | |
|---|---|---|---|---|---|---|---|
| NS = not significant | | | | | | | |

M402 had significant influence in reducing tumor take rate. Both 15 mg/kg and 30 mg/kg M402 significantly reduced the liver tumor size by 90% (p = 0.007) and 97% (p = 0.004) respectively and also were significantly more effective than 5FU/leucovorin (p = 0.041 and p = 0.011, respectively). Dalteparin (group 3) reduced liver tumor weight by approximately 81% (p = 0.017) when compared to the vehicle control group. Similarly, the cross-sectional area of the tumors also showed significant reduction with dalteparin (p = 0.027) and 15 and 30 mg/kg M402 (p = 0.016 and p = 0.004, respectively).

Mouse weights were monitored for the duration of the study. The mouse weights for each group remained within an acceptable range for all groups throughout the study.

### Model C: Breast cancer metastasis to the lung

M402 also showed anti-metastasis activity in a syngeneic orthotopic model of breast cancer metastasis (4T1).

Female BALB/c mice 8 weeks of age (WOA) were injected with 8x10⁴ 4T1 cell intra mammary fat pad. Daily treatment with saline or M402 with or without weekly treatment of cisplatin started on day 5. Primary tumors were removed on day 9 and weighed.

As shown in Table 2, cisplatin combined with M402 (10, 20, 30 mg/kg) showed a statistically significant decrease in lung metastasis compared to saline control group as determined by lung weight and tumor nodule counting (p < 0.05, One way ANOVA). Combination therapy groups (Cisplatin + M402 10/20/30 mg/kg) also had lower incidence of mammary tumor regrowth, thoracic cavity tumor metastasis, and weight loss (> 2 g) in the last 3 days before termination of the experiment. Combination therapy groups had higher incidence of transient weight loss (> 2 g) the week after surgery but recovered in one week.

**Table 2. 4T1 model: macroscopic tumor metastasis counts**

| **groups** | **# of mice** | **Lung tumor nodule # /animal** | **Average lung tumor size** | **Total tumor nodule** | **Average tumor size** | **Total tumor volume** |
|---|---|---|---|---|---|---|
| **Saline** | 15 | 6.0±4.7 | 1.4 | 90 | 2.0 | 122.10 |
| **Cisplatin** | 16 | 5.6±4.4 | 1.41 | 89 | 1.36 | 134.13 |
| **M402 30mg/kg** | 16 | 8.4±7.0 | 1.19 | 135 | 1.11 | 140.98 |
| **Cisplatin+M402 30mg/kg** | 16 | 3.1±3.7 | 0.88 | 49 | 0.85 | 28.78 |
| **Cisplatin+M402 20mg/kg** | 15 | 2.3+2.9 | 0.8 | 34 | 1.0 | 18.66 |
| **Cisplatin+M402 10mg/kg** | 16 | 2.3±2.5 | 1.41 | 37 | 1.41 | 57.39 |
| **untreated** | 7 | 12.9±14.0 | 0.98 | 90 | 1.3 | 65.06 |

In a second 4T1 experiment, female BALB/c mice 8 WOA were injected with 8x10⁴ 4T1 cells intra mammary fat pad. Continuous osmotic pump delivery of saline or M402 with weekly treatment of saline or Cisplatin started on day 4. Primary tumors were removed on day 9. There were no significant differences between the groups in primary tumor weight. However, immunohistology analyses showed significant decrease in microvessel density in tumors from mice treated with the combination of Cisplatin and M402. The experiment terminated on day 32 and different samples were taken. 6 mice were either found dead or were terminated early due to worsened overall condition.

4T1 lung metastases were determined by lung weight, lung tumor nodule quantification including nodule number, size and calculated tumor volume, as well as histological quantification. Results are shown in Figure 2. M402 (20 mg/kg/day) monotherapy groups did not significantly inhibit 4T1 lung metastasis. Cisplatin (1.25mg/kg) monotherapy showed significant anti-tumor efficacy (p<0.05). The combination of Cisplatin (1.25mg/kg) with M402 (20mg/kg/day) displayed efficacy in reducing lung metastasis (p<0.0005) and reducing microvessel density. Importantly, the combination therapy group also showed better anti-tumor efficacy when compared to the cisplatin monotherapy group determined by lung weight (p<0.02), tumor nodule number, lung tumor coverage by histology, and lung tumor microvessel density (p<0.05, t-test), demonstrating M402 enhanced the anti-tumor efficacy of cisplatin.

### Model D: Human prostate carcinoma PC-3M Model: combination therapy

Male SCID/Beige mice 8 WOA were injected with 5x10⁵ PC-3M-luciferase prostate carcinoma cells intra prostate. Daily treatment with saline or M402 with or without weekly treatment of cisplatin started on day 3. Mice were monitored weekly with Xenogen imaging system. The experiment was terminated on day 32. Different organs were isolated and tumor metastasis was assessed by weight and Xenogen imaging.

The M402 (30 mg/kg) monotherapy inhibited PC-3M metastasis in the peritoneum. Cisplatin combined with M402 (30 mg/kg) decreased tumor growth compared to saline and M402 monotherapy groups as determined by in vivo imaging. There was no significant difference between combination therapy (Cisplatin + M402 30 mg/kg) and Cisplatin monotherapy in primary tumor weight and metastasis under the specific experimental condition.

### Example 3: M402 treatment normalizes myeloid derived suppressor cells (MDSC), plasma MMP-9 and G-CSF level in 4T1 tumor-bearing mice

Groups of female BALB/c mice were inoculated orthotopically with 5x10⁴ 4T1 cells in the 4th mammary fat pad on day 0. Weekly ip injection of saline or Cisplatin (1.25mg/kg), and saline or M402 (20mg/kg) treatment by sc implanted osmotic pumps started on day 5. Primary tumors were removed on day 9 by surgery. Experiment terminated on day 32 and blood samples collected by cardiac puncture. 100ml of sodium citrate-treated pooled (by the cages) blood samples were treated with RBC lysis buffer, washed, and stained with different antibodies before analyzed with flow cytometry. Results are shown in Figure 5. (A) MDSC are defined by Forward and side scatter plot showing the expansion of these cells (more than 90% of these cells are CD11b+GR-1+) in 4T1 tumor-bearing mice. Quantification of these MDSC cells as % of total cells. *, P<0.05, **, P<0.01 when compared to saline control group. (B) Plasma samples were analyzed for MMP-9 concentration by Luminex system. *, P<0.05 compared to saline control group. (C) In a separate but similar experiment, plasma levels of G-CSF were determined by ELISA assay. **, P<0.01 compared to saline control group. As can be seen, M402 treatment normalized myeloid derived suppressor cells (MDSC), plasma MMP-9 and G-CSF level in 4T1 tumor-bearing mice.

### Example 4: M402 treatment increases monokine induced by gamma interferon (MIG) levels in 4T1 tumor-bearing mice

BALB/c mice were injected with saline or M402 at 20mg/kg twice daily for 7 days. Animals were sacrificed at different time point after the last dose and blood samples collected by cardiac puncture with sodium citrate as anticoagulant. Plasma was collected and stored in -80°C until MIG level determined using Luminex. As shown in Figure 7, M402 induced rapid increase in plasma MIG level, and the level gradually return to base line level.

### Example 5: M402 inhibits tumor cell mobilization and recruitment.

Results are shown in Figure 6. (A) Jurkat cells were added to the upper chambers of the 3 µm chemotaxis plates. Different concentrations of LMWH were combined with 3 ng/ml SDF-1α and added to the lower chamber in triplicates and incubated for 1 hr at 37°C after which migrated cells were quantified. M402 inhibited SDF-1α-induced Jurkat cell migration in a dose-dependent manner. (B) BALB/c mice were injected with saline or G-CSF in the presence or absence of M402 for 3 days. On the 4^{th} day, blood was analyzed for blood count by vetscan HM2. (C) Groups of female BALB/c mice were inoculated orthotopically with 5x10⁴ 4T1-luc-1A4 cells in the 4^{th} mammary fat pad. Saline or M402 (40 mg/kg/day) treatment by sc implanted osmotic pumps started on day 1. Primary tumors were removed on day 10 by surgery. Blood was collected on Day 30, at a time mice have significant metastatic tumor burden in the lung. RBC were lysed, blood was stained for MDSC (CD11b, Gr-1, CD45) before analysis by flow cytometry. M402 treatment significantly reduced MDSC in circulation. *, P<0.05, **, P<0.01 by ANOVA.

As can be seen in Figure 6, M402 inhibited tumor cell mobilization and recruitment.

## Claims

1. A method of monitoring the effect of a polysaccharide preparation on a subject having cancer, the method comprising determining tumor stromal levels of myeloid derived suppressor cells (MDSCs), endothelial progenitor cells (EPCs), plasma matrix metallopeptidase 9 (MMP-9) expression, granulocyte-colony stimulating factor (G-CSF) expression, monokine induced by gamma interferon (MIG) expression or combinations thereof in a sample obtained from the subject after the subject has been administered the polysaccharide preparation, to thereby determine the effect of the polysaccharide preparation on the subject;
wherein the polysaccharide preparation has the following characteristics:
a weight average chain molecular weight between 3,500 and 8,000 Da;
anti-Xa activity and anti-IIa activity each less than 50 IU/mg;
between 5% and 50% glycol split uronic acid residues; and
a molecular weight distribution such that 10-50% of the oligosaccharides of the preparation have a molecular weight < 3000 Da, 40-65% of the oligosaccharides have a molecular weight between 3000-8000 Da, and 5-30% of the oligosaccharides have a molecular weight > 8000 Da;
and wherein a decrease in the level of MDSCs, the level of EPCs, expression level of plasma MMP-9, expression level of G-CSF, or combinations thereof relative to a reference standard indicates that the polysaccharide preparation is effective in treating the cancer in the subject; and an increase or no significant change in the level of MDSCs, the level of EPCs, expression level of plasma MMP-9, expression level of G-CSF, or combinations thereof relative to a reference standard indicates that the polysaccharide preparation is not effective in treating the cancer in the subject.

2. The method of claim 1, wherein an increase in the expression level of MIG relative to a reference standard indicates that the polysaccharide preparation is effective in treating the cancer in the subject.

3. The method of claim 1, wherein a decrease or no significant change in the expression level of MIG relative to a reference standard indicates that the polysaccharide preparation is not effective in treating the cancer in the subject.

4. The method of any of the preceding claims, wherein the reference standard is MDSC levels, EPC levels, plasma MMP-9 expression levels, G-CSF expression levels and/or MIG expression levels in the subject prior to administration of the polysaccharide preparation.

5. The method of any of the preceding claims, wherein the sample is a blood or tissue sample.

6. The method of claim 5, wherein the sample is a biopsy sample.

7. The method of any of the preceding claims, wherein MDSC levels, EPC levels, plasma MMP-9 expression levels, G-CSF expression levels and/or MIG expression levels are determined by a method selected from the group consisting of enzyme-linked immunosorbent assay (ELISA), a radioimmunoassay (RIA), a Western blot, and an immunohistochemical assay (IHC).

8. The method of claim 1, wherein the polysaccharide preparation has previously been administered to the subject on one or more occasions.

9. The method of claim 1, wherein the polysaccharide preparation has between 5% and 30% glycol split uronic acid residues.

10. The method of claim 1, wherein the polysaccharide preparation has between 10% and 30% glycol split uronic acid residues.

11. The method of claim 1, wherein the polysaccharide chains of the polysaccharide preparation have no more than 3 glycol split uronic acid residues (U_{G}) per polysaccharide chain.

12. The method of claim 11, wherein each polysaccharide chain has no more than 2 or no more than 1 glycol split uronic acid residue (U_{G}) per polysaccharide chain.

13. The method of claim 11, wherein the polysaccharide chains of the polysaccharide preparation have on average no more than 3 glycol split uronic acid residues (U_{G}) per polysaccharide chain.

14. The method of claim 13, wherein the polysaccharide chains of the polysaccharide preparation have on average no more than 2 or no more than 1 glycol split uronic acid residues (U_{G}) per polysaccharide chain.

15. The method of claim 1, wherein the polysaccharide preparation has polysaccharide chains having greater than 40% U_{2S}H_{NS,6S} disaccharide residues.

16. The method of claim 15, wherein the polysaccharide preparation has greater than 70% U_{2S}H_{NS,6S} disaccharide residues.

17. The method of claim 16, wherein the polysaccharide preparation has a degree of desulfation less than 40%.

18. The method of claim 17, wherein the polysaccharide preparation has a degree of desulfation less than 30%.

19. The method of claim 18, wherein the polysaccharide preparation has a degree of desulfation less than 10%.

20. The method of claim 1, wherein the polysaccharide preparation comprises polysaccharides that include Formula I:
[U_{w}-H_{x,y,z}]ₘ~[U_{G}-H_{x,y,z}]ₙ
wherein each occurrence of U indicates a uronic acid residue and each occurrence of H indicates a hexosamine residue;
wherein m and n are integers such that
m = 4-16, and
n = 1-4;
each of w, x, y and z can independently be the same or different for each occurrence of [U_{w}-Hₓ,_{y,z}] and each of x, y and z can independently be the same or different for each occurrence of [U_{G}-H_{x,y,z}], wherein
w = -2OS or -20H;
x = -CNS or -NAc;
y = -3OS or -3OH;
z = -6OS or -60H;
and 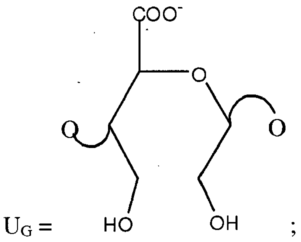
wherein the symbol - indicates that the units marked m and n are distributed along the polysaccharide chain and are not necessarily in sequence.

21. The method of claim 20, wherein n is 1-3.

22. The method of claim 1, wherein the cancer is selected from ovarian cancer, prostate cancer, lung cancer, liver cancer, breast cancer, glioma, gastric cancer, pancreatic cancer, head and neck cancer, colorectal cancer, lymphoma, multiple myeloma, melanoma, small intestinal cancer and thyroid carcinoma.

23. The method of claim 1, wherein the cancer is pancreatic cancer.

24. The method of claim 1, wherein the cancer is colorectal cancer.

25. The method of claim 1, wherein the cancer is a metastatic cancer.

26. The method of claim 25, wherein the metastatic cancer is metastatic pancreatic cancer.

27. The method of any of the preceding claims, the method comprising:
determining tumor stromal levels of MDSCs, EPCs, plasma MMP-9 expression, G-CSF expression, MIG expression or combinations thereof in a sample obtained from the subject prior to administration of the polysaccharide preparation; and
subsequently determining tumor stromal levels of MDSCs, EPCs, plasma MMP-9 expression, G-CSF expression, MIG expression or combinations thereof in a further sample obtained from the subject after the subject has been administered the polysaccharide preparation, to thereby determine the effect of the polysaccharide preparation on the subject.

28. The method of claim 1, wherein less than 30% of the uronic acid residues of the polysaccharide preparation are glycol split uronic acid residues.

29. The method of claim 1, wherein the polysaccharide preparation has an anti-Xa activity of less than 20 IU/mg, e.g. less than 10 IU/mg.

30. The method of claim 1, wherein the polysaccharide preparation has an anti-IIa activity of less than 20 IU/mg, e.g. less than 1 IU/mg.

31. The method of claim 1, wherein at least 50% of the chains in the polysaccharide preparation have a reducing end comprising a 2,5-anhydromannose residue that has been reduced to form an alcohol.

32. The method of claim 1, wherein the subject has been administered the polysaccharide preparation in combination with an additional therapy.

33. The method of claim 32, wherein the additional therapy is a chemotherapeutic agent.

34. The method of claim 33, wherein the chemotherapeutic agent is a cytotoxic agent selected from gemcitabine, taxanes and combinations thereof.

35. The method of claim 34, wherein the chemotherapeutic agent is gemcitabine.

## Patentansprüche

1. Verfahren zum Verfolgen der Wirkung eines Polysaccharidpräparats auf ein Individuum, das an Krebs leidet, wobei das Verfahren Folgendes umfasst:
Bestimmen des Tumorstromaspiegels der MDSCs (Myeloid Derived Suppressor Cells), der EPCs (Endothelial Progenitor Cells), der MMP-9-Expression (Plasma Matrix Metallopeptidase 9), der G-CSF-Expression (Granulocyte-Colony Stimulating Factor), der MIG-Expression (Monokine Induced by Gamma Interferon) oder Kombinationen davon in einer von dem Individuum stammenden Probe, nachdem das Polysaccharidpräparat dem Individuum verabreicht wurde, um dadurch die Wirkung des Polysaccharidpräparats auf das Individuum zu bestimmen;
wobei das Polysaccharidpräparat die folgenden Eigenschaften aufweist:
ein gewichtsmittleres Kettenmolekulargewicht zwischen 3500 und 8000 Da;
anti-Xa-Aktivität und anti-IIa-Aktivität von jeweils weniger als 50 IU/mg;
zwischen 5% und 50% durch Glycolspaltung erhaltende Uronsäurereste; und
solch eine Molekulargewichtsverteilung, dass 10-50% der Oligosaccharide des Präparats ein Molekulargewicht von < 3000 Da, 40-60% der Oligosaccharide ein Molekulargewicht zwischen 3000-8000 Da und 5-30% der Oligosaccharide ein Molekulargewicht von >8000 Da aufweisen;
und wobei ein Absinken des MDSCs-Spiegels, des EPCs-Spiegels, des Plasma-MMP-9-Expressionsspiegels, des G-CSF-Expressionsspiegels oder Kombinationen davon im Vergleich zu einem Referenzstandard anzeigt, dass das Polysaccharidpräparat wirksam für die Behandlung von Krebs in dem Individuum ist; und wobei ein Ansteigen oder keine wesentliche Veränderung des MDSCs-Spiegels, des EPCs-Spiegels, des Plasma-MMP-9-Expressionsspiegels, des G-CSF-Expressionsspiegels oder Kombinationen davon im Vergleich zu einem Referenzstandard angibt, dass das Polysaccharidpräparat nicht wirksam bei der Behandlung des Krebses in dem Individuum ist.

2. Verfahren nach Anspruch 1, wobei ein Ansteigen des MIG-Expressionsniveaus im Vergleich zu einem Referenzstandard anzeigt, dass das Polysaccharidpräparat wirksam für die Behandlung des Krebses in dem Individuum ist.

3. Verfahren nach Anspruch 1, wobei ein Absinken oder keine signifikante Veränderung des MIG-Expressionsniveaus im Vergleich zu einem Referenzstandard anzeigt, dass das Polysaccharidpräparat nicht wirksam für die Behandlung des Krebses in dem Individuum ist.

4. Verfahren nach einem der vorhergehenden Ansprüche, wobei es sich bei dem Referenzstandard um MDSC-Spiegel, EPC-Spiegel, Plasma-MMP-9-Expressionsspiegel, G-CSF-Expressionsspiegel und/oder MIG-Expressionsspiegel in dem Individuum vor der Verabreichung des Polysaccharidpräparats handelt.

5. Verfahren nach einem der vorhergehenden Ansprüche, wobei es sich bei der Probe um eine Blut- oder Gewebeprobe handelt.

6. Verfahren nach Anspruch 5, wobei es sich bei der Probe um eine Biopsieprobe handelt.

7. Verfahren nach einem der vorhergehenden Ansprüche, wobei die MDSC-Spiegel, EPC-Spiegel, Plasma-MMP-9-Expressionsspiegel, G-CSF-Expressionsspiegel und/oder MIG-Expressionsspiegel mit einem Verfahren, ausgewählt aus der Gruppe bestehend aus enzymgebundenem Immunsorptionsassay (ELISA), einem Radioimmunassay (RIA), einem Western-Blot und einem immunhistochemischen Assay (IHC) bestimmt werden.

8. Verfahren nach Anspruch 1, wobei das Polysaccharidpräparat dem Individuum zuvor ein- oder mehrmals verabreicht worden ist.

9. Verfahren nach Anspruch 1, wobei das Polysaccharidpräparat zwischen 5% und 30% durch Glycolspaltung erhaltene Uronsäurereste aufweist.

10. Verfahren nach Anspruch 1, wobei das Polysaccharidpräparat zwischen 10% und 30% durch Glycolspaltung erhaltene Uronsäurereste aufweist.

11. Verfahren nach Anspruch 1, wobei die Polysaccharidketten des Polysaccharidpräparats nicht mehr als 3 durch Glycolspaltung erhaltene Uronsäurereste (U_{G}) pro Polysaccharidkette aufweisen.

12. Verfahren nach Anspruch 11, wobei jede Polysaccharidkette nicht mehr als 2 durch Glycolspaltung erhaltene Uronsäurereste (U_{G}) oder nicht mehr als 1 durch Glycolspaltung erhaltenen Uronsäurerest (U_{G}) pro Polysaccharidkette aufweist.

13. Verfahren nach Anspruch 11, wobei die Polysaccharidketten des Polysaccharidpräparats durchschnittlich nicht mehr als 3 durch Glycolspaltung erhaltene Uronsäurereste (U_{G}) pro Polysaccharidkette aufweisen.

14. Verfahren nach Anspruch 13, wobei die Polysaccharidketten des Polysaccharidpräparats durchschnittlich nicht mehr als 2 durch Glycolspaltung erhaltene Uronsäurereste (U_{G}) oder nicht mehr als 1 durch Glycolspaltung erhaltenen Uronsäurerest (U_{G}) pro Polysaccharidkette aufweisen.

15. Verfahren nach Anspruch 1, wobei das Polysaccharidpräparat mehr als 40% U_{2S}H_{NS,6S}-Disaccharidreste aufweistt.

16. Verfahren nach Anspruch 15, wobei das Polysaccharidpräparat mehr als 70% U_{2S}H_{NS,6S}-Disaccharidreste aufweist.

17. Verfahren nach Anspruch 16, wobei das Polysaccharidpräparat einen Desulfatierungsgrad von weniger als 40% aufweist.

18. Verfahren nach Anspruch 17, wobei das Polysaccharidpräparat einen Desulfatierungsgrad von weniger als 30% aufweist.

19. Verfahren nach Anspruch 18, wobei das Polysaccharidpräparat einen Desulfatierungsgrad von weniger als 10% aufweist.

20. Verfahren nach Anspruch 1, wobei das Polysaccharidpräparat Polysaccharide, die Formel I beinhalten, umfasst:
[U_{w}-H_{x, y, z}]ₘ ~ [U_{G}-Hₓ,_{y,z}]ₙ;
wobei jedes Vorkommen von U einen Uronsäurerest angibt und jedes Vorkommen von H einen Hexosaminrest angibt;
wobei m und n solche ganzen Zahlen sind, dass
m = 4-16 und
n = 1-4;
wobei jedes von w, x, y und z unabhängig für jedes Vorkommen von [U_{w}-H_{x,y,z}] gleich oder verschieden sein kann und jedes von x, y und z unabhängig für jedes Vorkommen von [U_{G}-H_{x,y,z}] gleich oder verschieden sein kann, wobei
w = -20S oder -20H;
x = -NS oder -NAc;
y = -3OS oder -30H;
y = -6OS oder -60H;
und 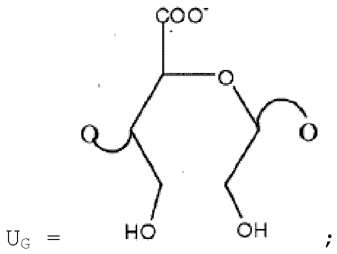
wobei das Symbol - angibt, dass die mit m und n bezeichneten Einheiten entlang der Polysaccharidkette verteilt und nicht zwingenderweise in dieser Reihenfolge sind.

21. Verfahren nach Anspruch 20, wobei n 1-3 beträgt.

22. Verfahren nach Anspruch 1, wobei der Krebs aus der Reihe Eierstockkrebs, Prostatakrebs, Lungenkrebs, Leberkrebs, Brustkrebs, Gliom, Magenkrebs, Bauspeicheldrüsenkrebs, Kopf- und Halskrebs, Kolorektalkrebs, Lymphom, multiples Myelom, Melanom, Dünndarmkrebs und Schildrüsenkarzinom ausgewählt ist.

23. Verfahren nach Anspruch 1, wobei es sich bei dem Krebs um Bauchspeicheldrüsenkrebs handelt.

24. Verfahren nach Anspruch 1, wobei es sich bei dem Krebs um Kolorektalkrebs handelt.

25. Verfahren nach Anspruch 1, wobei es sich bei dem Krebs um einen metastasierenden Krebs handelt.

26. Verfahren nach Anspruch 25, wobei es sich bei dem metastasierenden Krebs um metastasierenden Bauchspeicheldrüsenkrebs handelt.

27. Verfahren nach einem der vorhergehenden Ansprüche, wobei das Verfahren Folgendes umfasst:
Beurteilen der Tumorstromaspiegel der MDSCs, EPCs, Plasma-MMP-9-Expression, G-CSF-Expression, MIG-Expression oder von Kombinationen davon in einer Probe, die von dem Individuum vor Verabreichung des Polysaccharidpräparats stammt; und
anschließendes Beurteilen der Tumorstromaspiegel der MDSCs, EPCs, Plasma-MMP-9-Expression, G-CSF-Expression, MIG-Expression oder von Kombinationen davon in einer weiteren Probe, die von dem Individuum stammt, nachdem dem Individuum das Polysaccharidpräparat verabreicht wurde, um dadurch die Wirkung des Polysaccharidpräparats auf das Individuum zu bestimmen.

28. Verfahren nach Anspruch 1, wobei es sich bei weniger als 30% der Uronsäurereste des Polysaccharidpräparats um durch Glycolspaltung erhaltene Uronsäurereste handelt.

29. Verfahren nach Anspruch 1, wobei das Polysaccharidpräparat eine anti-Xa-Aktivität von weniger als 20 IU/mg, z.B. weniger als 10 IU/mg, aufweist.

30. Verfahren nach Anspruch 1, wobei das Polysaccharidpräparat eine anti-IIa-Aktivität von weniger als 20 IU/mg, z.B. weniger als 1IU/mg, aufweist.

31. Verfahren nach Anspruch 1, wobei mindestens 50% der Ketten in dem Polysaccharidpräparat ein reduzierendes Ende, umfassend einen 2,5-Anhydromannoserest, der zu einem Alkohol reduziert worden ist, aufweisen.

32. Verfahren nach Anspruch 1, wobei dem Individuum das Polysaccharidpräparat in Kombination mit einer zusätzlichen Therapie verabreicht worden ist.

33. Verfahren nach Anspruch 32, wobei es sich bei der zusätzlichen Therapie um ein Chemotherapeutikum handelt.

34. Verfahren nach Anspruch 33, wobei es sich bei dem Chemotherapeutikum um ein cytotoxisches Mittel aus der Reihe Gemcitabin, Taxane und Kombinationen davon handelt.

35. Verfahren nach Anspruch 34, wobei es sich bei dem Chemotherapeutikum um Gemcitabin handelt.

## Revendications

1. Procédé pour surveiller l'efficacité d'une préparation polysaccharidique sur un sujet ayant un cancer, le procédé comprenant la détermination des niveaux du stroma tumoral de cellules myéloïdes suppressives (MDSC), de cellules progénitrices endothéliales (EPC), de l'expression de la métallopeptidase 9 de la matrice plasmatique (MMP-9), de l'expression du facteur de stimulation des colonies de granulocytes (G-CSF), de l'expression des monokines induites par l'interféron gamma (MIG), ou des combinaisons de celles-ci, dans un échantillon obtenu du sujet après que le sujet a reçu en administration la préparation polysaccharidique, pour déterminer ainsi l'effet de la préparation polysaccharidique sur le sujet ;
dans lequel la préparation polysaccharidique a les caractéristiques suivantes :
une masse moléculaire moyenne en masse de la chaîne comprise entre 3500 et 8000 Da ;
une activité anti-Xa et une activité anti-IIa, chacune inférieure à 50 UI/mg ;
entre 5 et 50 % de résidus d'acide uronique dissociés au glycol ; et
une distribution des masses moléculaires telle que 10-50 % des oligosaccharides de la préparation aient une masse moléculaire < 3000 Da, 40-65 % des oligosaccharides aient une masse moléculaire comprise entre 3000 et 8000 Da, et 5-30 % des oligonucléotides aient une masse moléculaire > 8000 Da ;
et dans lequel une diminution du niveau des MDSC, du niveau des EPC, du niveau d'expression de la MMP-9 plasmatique, du niveau d'expression du G-CSF, ou des combinaisons de celles-ci, par comparaison avec un étalon de référence, indique que la préparation polysaccharidique est efficace pour ce qui est de traiter le cancer chez le sujet ; et une augmentation ou un changement non significatif du niveau des MDSC, du niveau des EPC, du niveau d'expression de la MMP-9 plasmatique, du niveau d'expression du G-CSF, ou des combinaisons de celles-ci, par comparaison avec un étalon de référence, indique que la préparation polysaccharidique n'est pas efficace pour ce qui est de traiter le cancer chez le sujet.

2. Procédé selon la revendication 1, dans lequel une augmentation du niveau d'expression de la MIG par comparaison avec un étalon de référence indique que la préparation polysaccharidique est efficace pour ce qui est de traiter le cancer chez le sujet.

3. Procédé selon la revendication 1, dans lequel une diminution ou un changement non significatif du niveau d'expression de la MIG par comparaison avec un étalon de référence indique que la préparation polysaccharidique n'est pas efficace pour ce qui est de traiter le cancer chez le sujet.

4. Procédé selon l'une quelconque des revendications précédentes, dans lequel l'étalon de référence est les niveaux de MDSC, les niveaux d'EPC, les niveaux d'expression de la MMP-9 plasmatique, les niveaux d'expression du G-CSF et/ou les niveaux d'expression de la MIG chez le sujet avant administration de la préparation polysaccharidique.

5. Procédé selon l'une quelconque des revendications précédentes, dans lequel l'échantillon est un échantillon sanguin ou tissulaire.

6. Procédé selon la revendication 5, dans lequel l'échantillon est un échantillon biopsique.

7. Procédé selon l'une quelconque des revendications précédentes, dans lequel les niveaux de MDSC, les niveaux d'EPC, les niveaux d'expression de la MMP-9 plasmatique, les niveaux d'expression du G-CSF et les niveaux d'expression de la MIG sont déterminés par une méthode choisie dans le groupe consistant en un essai par immunosorption à liaison enzymatique (ELISA), un radioimmuno-essai (RIA), un transfert de Western, et un essai immunohistochimique (IHC).

8. Procédé selon la revendication 1, dans lequel la préparation polysaccharidique a été préalablement administrée au sujet à une ou plusieurs occasions.

9. Procédé selon la revendication 1, dans lequel la préparation polysaccharidique contient de 5 à 30 % de résidus d'acide uronique dissociés au glycol.

10. Procédé selon la revendication 1, dans lequel la préparation polysaccharidique contient 10 à 30 % de résidus d'acide uronique dissociés au glycol.

11. Procédé selon la revendication 1, dans lequel les chaînes polysaccharidiques de la préparation polysaccharidique ne possèdent pas plus de 3 résidus d'acide uronique dissociés au glycol (U_{G}) par chaîne polysaccharidique.

12. Procédé selon la revendication 11, dans lequel chaque chaîne polysaccharidique ne possède pas plus de 2 ou pas plus de 1 résidus d'acide uronique dissociés au glycol (U_{G}) par chaîne polysaccharidique.

13. Procédé selon la revendication 11, dans lequel les chaînes polysaccharidiques de la préparation polysaccharidique contiennent en moyenne pas plus de 3 résidus d'acide uronique dissociés au glycol (U_{G}) par chaîne polysaccharidique.

14. Procédé selon la revendication 13, dans lequel les chaînes polysaccharidiques de la préparation polysaccharidique contiennent en moyenne pas plus de 2 ou pas plus de 1 résidus d'acide uronique dissociés au glycol (U_{G}) par chaîne polysaccharidique.

15. Procédé selon la revendication 1, dans lequel la préparation polysaccharidique contient des chaînes polysaccharidiques ayant plus de 40 % de résidus disaccharidiques U_{2S}H_{NS,6S}.

16. Procédé selon la revendication 15, dans lequel la préparation polysaccharidique contient plus de 70 % de résidus disaccharidiques U_{2S}H_{NS,65}.

17. Procédé selon la revendication 16, dans lequel la préparation polysaccharidique a un degré de désulfatation inférieur à 40 %.

18. Procédé selon la revendication 17, dans lequel la préparation polysaccharidique a un degré de désulfatation inférieur à 30 %.

19. Procédé selon la revendication 18, dans lequel la préparation polysaccharidique a un degré de désulfatation inférieur à 10 %.

20. Procédé selon la revendication 1, dans lequel la préparation polysaccharidique comprend des polysaccharides qui comprennent la Formule I :
**[U_{w}-H_{x,y,z}]ₘ∼[U_{G}-H_{x,y,z}]ₙ**
dans laquelle chaque occurrence de U indique un résidu d'acide uronique et chaque occurrence de H indique un résidu d'hexosamine ;
dans laquelle m et n sont des entiers tels que
m = 4-16 et
n = 1-4 ;
chacun de w, x, y et z peut indépendamment être identique ou différent pour chaque occurrence de [U_{w}-H_{x,y,z}], et chacun de x, y et z peut indépendamment être identique ou différent pour chaque occurrence de [U_{G}-H_{x,y,z}], où
w = -20S ou -2OH ;
x = -NS ou -NAc ;
y = -3OS ou -3OH ;
z = -6OS ou -6OH ;
et 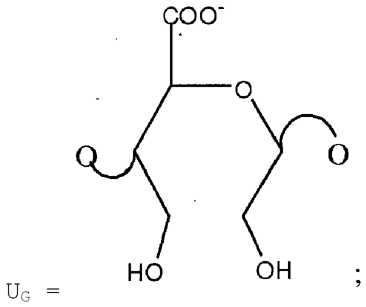 où le symbole ∼ indique que les unités marquées m et n sont distribuées le long de la chaîne polysaccharidique et ne sont pas nécessairement successives.

21. Procédé selon la revendication 20, dans lequel n = 1-3.

22. Procédé selon la revendication 1, dans lequel le cancer est choisi parmi le cancer des ovaires, le cancer de la prostate, le cancer du poumon, le cancer du foie, le cancer du sein, le gliome, le cancer gastrique, le cancer du pancréas, le cancer de la tête et du cou, le cancer colorectal, le lymphome, le myélome multiple, le mélanome, le cancer de l'intestin grêle et le cancer de la thyroïde.

23. Procédé selon la revendication 1, dans lequel le cancer est le cancer du pancréas.

24. Procédé selon la revendication 1, dans lequel le cancer est le cancer colorectal.

25. Procédé selon la revendication 1, dans lequel le cancer est un cancer métastatique.

26. Procédé selon la revendication 25, dans lequel le cancer métastatique est le cancer métastatique du pancréas.

27. Procédé selon l'une quelconque des revendications précédentes, le procédé comprenant :
la détermination des niveaux de stroma tumoral des MDSC, des EPC, de l'expression de la MMP-9 plasmatique, de l'expression du G-CSF, de l'expression de la MIG ou de combinaisons de ceux-ci, dans un échantillon obtenu du sujet avant administration de la préparation polysaccharidique ; et
puis la détermination des niveaux de stroma tumoral des MDSC, des EPC, de l'expression de la MMP-9 plasmatique, de l'expression du G-CSF, de l'expression de la MIG ou de combinaisons de ceux-ci dans un autre échantillon obtenu du sujet après que le sujet a reçu en administration la préparation polysaccharidique, pour déterminer ainsi l'effet de la préparation polysaccharidique sur le sujet.

28. Procédé selon la revendication 1, dans lequel moins de 30 % des résidus d'acide uronique de la préparation polysaccharidique sont des résidus d'acide uronique dissociés au glycol.

29. Procédé selon la revendication 1, dans lequel la préparation polysaccharidique a une activité anti-Xa inférieure à 20 UI/mg, p.ex. inférieure à 10 UI/mg.

30. Procédé selon la revendication 1, dans lequel la préparation polysaccharidique a une activité anti-IIa inférieure à 20 UI/mg, p.ex. inférieure à 1 UI/mg.

31. Procédé selon la revendication 1, dans lequel au moins 50 % des chaînes de la préparation polysaccharidique ont une extrémité réductrice comprenant un résidu de 2,5-anhydromannose qui a été réduit pour former un alcool.

32. Procédé selon la revendication 1, dans lequel le sujet a reçu en administration la préparation polysaccharidique en combinaison avec un traitement additionnel.

33. Procédé selon la revendication 32, dans lequel le traitement additionnel est un agent chimiothérapeutique.

34. Procédé selon la revendication 33, dans lequel l'agent chimiothérapeutique est un agent cytotoxique choisi parmi la gemcitabine, les taxanes et les combinaisons de ces derniers.

35. Procédé selon la revendication 34, dans lequel l'agent chimiothérapeutique est la gemcitabine.
